(19) Europäisches Patentamt
European Patent Office
Office européen des brevets

(11) EP 2 722 352 A1

(12) EUROPEAN PATENT APPLICATION
published in accordance with Art. 153(4) EPC

(43) Date of publication:
**23.04.2014 Bulletin 2014/17**

(21) Application number: **12800743.2**

(22) Date of filing: **15.06.2012**

(51) Int Cl.:
***C08G 63/183*** *(2006.01)* ***C07C 69/82*** *(2006.01)*

(86) International application number:
**PCT/JP2012/065320**

(87) International publication number:
**WO 2012/173220 (20.12.2012 Gazette 2012/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.06.2011 JP 2011135019**
**17.06.2011 US 201161498166 P**

(71) Applicants:
- **Toray Industries, Inc.**
  **Tokyo 103-8666 (JP)**
- **Gevo, Inc.**
  **Centennial, Colorado 80112 (US)**

(72) Inventors:
- **TANAKA, Youichiro**
  **Shizuoka 4118652 (JP)**
- **MORIMOTO, Kunihiro**
  **Otsu-shi**
  **Shiga 520-8558 (JP)**
- **OKUBO, Takuro**
  **Nagoya-shi**
  **Aichi 455-8502 (JP)**
- **MURATA, Yoshiaki**
  **Nagoya-shi**
  **Aichi 455-8502 (JP)**
- **PETERS W. Matthew**
  **Colorado 80112 (US)**

(74) Representative: **Webster, Jeremy Mark et al**
**Mewburn Ellis LLP**
**33 Gutter Lane**
**London**
**EC2V 8AS (GB)**

# (54) METHOD FOR MANUFACTURING BIOMASS-DERIVED POLYESTER AND BIOMASS-DERIVED POLYESTER

(57) [Problem] To provide a method of producing a biomass resource-derived polyester capable of greatly reducing fossil resource usage amounts and carbon dioxide increases that is in way inferior in color or thermal stability to a conventional fossil resource-derived product while also having superior dye affinity.

[Solution] The biomass resource-derived dicarboxylic acid and/or ester forming derivative thereof used as the raw material fulfills at least one of the following conditions:

(A) The amount of potassium hydroxide needed to neutralize an acid component extracted using an organic solvent from 1 g of the dicarboxylic acid and/or ester forming derivative thereof is 0.1 mg KOH/g or less

(B) The amount of potassium hydroxide needed to neutralize an acid component extracted using water from 1 g of the dicarboxylic acid and/or ester forming derivative thereof is 0.05 mg KOH/g or less

(C) Sulfuric acid ion content is 40 ppm or less

EP 2 722 352 A1

## Description

### Technical field

The present invention relates to a method of producing a polyester obtained from a biomass resource-derived material and a biomass resource-derived polyester.

### Background art

The fossil resource petroleum is an important resource for the chemical industries; however, due to the large amounts of carbon dioxide emitted during manufacturing processes and incineration, on top of the existing concerns about petroleum sources drying up, it has also led to environmental problems such as global-scale warming. Against such circumstances, much attention is being devoted to the use of renewable materials and materials placing a low burden on the environment.

Biomass resources are created by the conversion of the raw materials of water and carbon dioxide during photosynthesis in plants, and include starch, carbohydrates, cellulose, lignin, and the like. Because the biomass resource production process utilizes carbon dioxide as a raw material, materials using biomass resources generate no new carbon dioxide even when broken down into water and carbon dioxide by incineration after being used, and, in some cases, the carbon dioxide is reabsorbed by plants, making such materials renewable. If it were possible to use such biomass resources in place of fossil resources, reductions in fossil resources and increases in carbon dioxide would be controlled.

Meanwhile, polyester is one of the most widely used synthetic resins in the world due to its superior mechanical strength, chemical stability, and transparency, as well as its low cost, and is used in diverse types of fiber, film, sheet, and bottle. Many attempts to synthesize polyester, which is thus so widely used, from renewable biomass resources have been made. For example, fermenting corn to obtain 1,3-propanediol (1,3-PDO) through biological and chemical engineering processes, which is used in polypropylene terephthalate (PPT) containing biomass material derived from non-fossil resources and polyethylene terephthalate (PET) using biomass resource-derived ethylene glycol, has been reported (patent documents 1-4). However, the theoretical bio-based content as determined from the $^{14}C$ concentrations of these polymers is only 27% (PPT) and 20% (PET). As opposed to this, polybutylene terephthalate (patent document 5) and polyethylene terephthalate (patent document 6) having a bio-based content using a biomass resource-derived terephthalate component of 94% have also been reported; however, these have the problems that, because various biomass resource-derived trace impurities derived from amino acids, proteins, metallic cations, and the like are present in biomass resource-derived polyester materials, polymerization reactivity is poor and it is difficult to obtain a polymer of a viscosity sufficient to withstand applied use; and even if such a polymer could be obtained, coloration would be dramatic, drastically limiting potential uses. In order to solve these problems, the inventors' research group reported that a biomass resource-derived polyester with a high bio-based content and exhibiting good polymerization reactivity and polymer color could be obtained by including a phosphorous compound in the biomass resource-derived polyester (patent document 7). However, color and thermal stability remained inferior to fossil resource-derived polyesters obtained under the same conditions. In order to substitute biomass resource-derived polyester products for conventional fossil resource-derived polyester products, there was a demand to obtain a color tone equivalent to that of fossil resource-derived polyester at the polyester pellet stage. In particular, there was a demand for color equivalent to that of conventional fossil resource-derived products in the pellet stage in the case of fibers upon which dyeing or the like is performed, because a color different from that of conventional products in the polyester pellet stage yields a different texture from that of conventional products after spinning and dyeing. However, a biomass resource-derived polyester meeting these demands has not yet been obtained.

### Prior art documents

#### Patent documents

[Patent Document 1] U.S. Patent 6,428,767 B1 (Examples)
[Patent Document 2] Chinese Patent Publication CN101046007 (Claims)
[Patent Document 3] Unexamined Japanese Patent Application Publication 2009-91694 (Claims)
[Patent Document 4] PCT Publication WO2009072462 (Claims)
[Patent Document 5] PCT Publication WO2010078328 (Claims)
[Patent Document 6] PCT Publication WO2009120457 (Claims)
[Patent Document 7] Unexamined Japanese Patent Application Publication 2011-219736 (Claims)

## Summary of the invention

### Problem to be solved by the invention

The present invention seeks to provide a method of producing a biomass resource-derived polyester having a color and thermal stability such that substitution for conventional fossil resource-derived polyester products is possible as well as a biomass resource-derived polyester obtained by means of the same, as these enable reductions in fossil resources and increases in carbon dioxide to be greatly controlled.

### Means of solving the problem

As the result of diligent research into a solution for the problems described above, it was discovered that, in a method of producing a biomass resource-derived polyester using a biomass resource-derived dicarboxylic acid and/or ester forming derivative thereof, these problems could be solved by setting at least one of the organic acid component content, inorganic acid component content, or sulfuric acid ion content of the biomass resource-derived dicarboxylic acid and/or ester forming derivative thereof within a fixed range.

Specifically, the method of producing a biomass resource-derived polyester according to the present invention is characterized in that the biomass resource-derived dicarboxylic acid and/or ester forming derivative thereof fulfills at least one of the conditions (A), (B), and (C) described below.

(A) The amount of potassium hydroxide needed to neutralize the acid component extracted using an organic solvent from 1 g of the dicarboxylic acid and/or ester forming derivative thereof is 0.1 mg KOH/g or less
(B) The amount of potassium hydroxide needed to neutralize the acid component extracted using water from 1 g of the dicarboxylic acid and/or ester forming derivative thereof is 0.05 mg KOH/g or less
(C) The sulfuric acid ion content is 40 ppm or less

The method of producing a biomass resource-derived polyester according to the present invention is also characterized in using a biomass resource-derived dicarboxylic acid and/or ester forming derivative thereof obtained by a process including the steps (1) through (4) described below.

(1) Obtaining isobutanol from a biomass resource by means of a fermentation method
(2) Obtaining isobutene from the isobutanol by means of dehydration
(3) Converting the isobutene to paraxylene
(4) Obtaining a terephthalic acid and/or ester forming derivative thereof from the paraxylene

The biomass resource-derived dicarboxylic acid and/or ester forming derivative thereof is further characterized in fulfilling at least one of the conditions (A), (B), and (C) described above. The method of producing a biomass resource-derived dicarboxylic acid and/or ester forming derivative thereof according to the present invention is also characterized in that a dialkyl ester of the biomass resource-derived dicarboxylic acid is refined by distillation.

### Effect of the invention

In accordance with the production method according to the present invention, it is possible to obtain a biomass resource-derived polyester having superior polymerization reactivity and a viscosity high enough for practical application, as well as color and thermal stability good enough that substitution for a conventional fossil resource-derived polyester is possible, by performing polymerization using a biomass resource-derived dicarboxylic acid and/or ester forming derivative thereof fulfilling at least one of the conditions (A), (B), and (C) described above.

It is preferable that the biomass resource-derived dicarboxylic acid and/or ester forming derivative thereof used in the production method according to the present invention have an ash content of 100 ppm or less. It is also preferable that a biomass resource-derived diol be used as a raw material along with the biomass resource-derived dicarboxylic acid and/or ester forming derivative thereof described above, allowing the bio-based content to be further increased. It is also preferable that an antioxidant be used.

The intrinsic viscosity of the biomass resource-derived polyester obtained using the production method according to the present invention intrinsic viscosity is preferably from 0.4 to 2.0 $dlg^{-1}$. The color value b of the polyester is preferably from -10 to 15. Furthermore, the diethylene glycol content of the polyester is preferable from 0.1 to 3.0% by weight.

Not only can the biomass resource-derived dicarboxylic acid and/or ester forming derivative thereof fulfilling at least one of the conditions (A) through (C) described above be favorably used as a raw material for producing the biomass

resource-derived polyester, it can also be turned to a wide variety of other uses. It is preferable that the biomass resource-derived dicarboxylic acid and/or ester forming derivative thereof have an ash content of 100 ppm or less. Such a biomass resource-derived dicarboxylic acid and/or ester forming derivative thereof can be easily obtained by distillation refining, preferably by distillation refining under alkaline conditions.

The biomass resource-derived polyester obtained by means of the present invention can be substituted for conventional fossil resource-derived polyesters, which are currently used in great amounts for a wide variety of uses, allowing reductions in fossil resources and increases in carbon dioxide to be greatly controlled.

Description of embodiments

In the method of producing a biomass resource-derived polyester according to the present invention, it is essential that the biomass resource-derived dicarboxylic acid and/or ester forming derivative thereof be used as a dicarboxylic acid component. By using the biomass resource-derived dicarboxylic acid and/or ester forming derivative thereof as a dicarboxylic acid component, it is possible to achieve the objects of increasing the bio-based content, reducing the amount of fossil resources used, and controlling increases in carbon dioxide. The ratio of the biomass resource-derived dicarboxylic acid and/or ester forming derivative thereof to the total dicarboxylic acid component content is preferably 80 mol% or more, and more preferably 100 mol%.

However, traces amounts of impurities, such as alkaline components or metal cations having Lewis acid properties, derived from biomass resource-derived amino acids, proteins, and the like are present in the raw materials for synthesizing the biomass resource-derived dicarboxylic acid and/or ester forming derivative thereof. For this reason, it is not possible to obtain the biomass resource-derived dicarboxylic acid and/or ester forming derivative thereof using a method similar to one used for fossil resource-derived compounds due to a reduction in reactivity caused by these impurities, so that excessive amounts of catalysts and other reagents become necessary compared to when synthesizing an equivalent fossil resource-derived compound, or it becomes necessary to use large amounts of inorganic acid during the intermediate steps or repeat the step of obtaining dialkyl ester from the dicarboxylic acid; as a result, the obtained biomass resource-derived dicarboxylic acid and/or ester forming derivative thereof contains large amounts of the inorganic acid component or sulfuric acid ions used in the intermediate steps, along with biomass resource-derived organic acids or organic acids resulting from incomplete reaction. The presence of these organic acid components, inorganic acid components, and sulfuric acid ions increase the amount of diethylene glycol byproduct, not only lowering the melting point of the obtained polyester and damaging its thermal stability but also negatively affecting the color of the polyester.

It is preferable that the biomass resource-derived dicarboxylic acid and/or ester forming derivative thereof according to the present invention be obtained according to a production method including a refining step. Examples of refining methods include methods using ion chromatography, distillation, recrystallization, and sublimation as well as crystallization using water, alcohol, carboxylic acid, or a mixture thereof, or a desired combination of desired unit processes such as washing, filtration, desiccation, and the like, repeated as necessary. Through such refining methods, it is possible to obtain a biomass resource-derived dicarboxylic acid and/or ester forming derivative thereof suitable for the present invention. Of these, distillation refining a dialkyl ester of the biomass resource-derived dicarboxylic acid is industrially simple and is preferable, and distillation refining under alkaline conditions is especially preferable, and it is possible to obtain a biomass resource-derived dicarboxylic acid and/or ester forming derivative thereof perform having a limited organic acid component and/or inorganic acid component and/or sulfuric acid ion content while performing highly efficient refining and maintaining a high recovery rate.

In the context of the present invention, "distillation refining under alkaline conditions" refers to distilling with an alkaline substance copresent. The alkaline substance is not particular limited, but is preferably an inorganic alkaline substance such as sodium hydroxide, potassium hydroxide, sodium carbonate, potassium carbonate, sodium hydrogen carbonate, potassium bicarbonate, or the like; and more preferably sodium carbonate or potassium carbonate.

There is also no particular limitation upon the amount of alkaline substance copresent, and, for example, an amount equal to or greater than the amount of organic acid component and/or inorganic acid component and/or sulfuric acid ion contained by the biomass resource-derived dicarboxylic acid and/or ester forming derivative thereof.

The biomass resource-derived dicarboxylic acid and/or ester forming derivative thereof according to the present invention is characterized in fulfilling at least one of the conditions (A), (B), and (C) described below.

(A) The amount of potassium hydroxide needed to neutralize the acid component extracted using an organic solvent from 1 g of the dicarboxylic acid and/or ester forming derivative thereof is 0.1 mg KOH/g or less
(B) The amount of potassium hydroxide needed to neutralize the acid component extracted using water from 1 g of the dicarboxylic acid and/or ester forming derivative thereof is 0.05 mg KOH/g or less
(C) The sulfuric acid ion content is 40 ppm or less

In the present invention, it is necessary that the biomass resource-derived dicarboxylic acid and/or ester forming

derivative thereof fulfill at least one of the conditions (A), (B), and (C) described above; one condition may be fulfilled, or two or all three may be fulfilled. The conditions (A) through (C) will be described below.

In the present invention, it is preferable that (A) the amount of potassium hydroxide needed to neutralize the acid component extracted using an organic solvent from 1 g of the biomass resource-derived dicarboxylic acid and/or ester forming derivative thereof be 0.1 mg KOH/g or less. If the amount of potassium hydroxide required for neutralization exceeds 0.1 mg KOH/g, polymerization reactivity decreases, and coloration or a reduction in thermal stability of the obtained polyester occurs. It is preferable that as small an amount as possible of potassium hydroxide necessary for neutralization be used, but it is not realistic to reduce the amount to less than 0.0001 mg KOH/g as this leads to excessive costs during the refining step; moreover, if small amounts of acid component remain present, there is a slight increase in the byproduction of diethylene glycol especially during polyethylene terephthalate polymerization, improving the dye affinity of the obtained fiber without damaging the color or thermal stability of the obtained polyester. The amount is preferably within a range from 0.0001 to 0.1 mg KOH/g, more preferably from 0.0005 to 0.08 mg KOH/g, and especially preferably from 0.001 to 0.05 mg KOH/g.

The organic solvent used in condition (A) of the present invention can be any solvent capable of extracting the organic acid component, such as a dicarboxylic acid monoalkyl ester or biomass resource-derived amino acid constituting an impurity within the ester forming derivative of the biomass resource-derived dicarboxylic acid; examples include alcohol solvents such as methanol, ethanol, n-propanol, isopropanol, n-butanol, sec-butanol, ethylene glycol, 1,3-propanediol, 1,4-butanediol, and the like; and aromatic hydrocarbon solvents such as benzene, toluene, o-xylene, p-xylene, m-xylene, and the like; ether solvents such as diethyl ether, dibutyl ether, tetrahydrofuran, 1,4-dioxane, and the like; as well as ethyl acetate, acetonitrile, acetone, dimethyl formamide, dimethyl sulfoxide, and the like. These may be used singly or in combinations of two or more types. Of these, it is especially preferable that a mixture of ethanol and paraxylene at a 1:2 ratio be used as the organic solvent.

In the present invention, it is preferable that (B) the amount of potassium hydroxide needed to neutralize the acid component extracted using water from 1 g of the biomass resource-derived dicarboxylic acid and/or ester forming derivative thereof be 0.05 mg KOH/g or less. If the amount of potassium hydroxide required for neutralization exceeds 0.05 mg KOH/g, the color and thermal stability of the obtained polyester are negatively affected. It is preferable that as small an amount as possible of potassium hydroxide necessary for neutralization be used, but it is not realistic to reduce the amount to less than 0.0001 mg KOH/g as this leads to excessive costs during the refining step; moreover, if small amounts of acid component remain present, there is a slight increase in the byproduction of diethylene glycol especially during polyethylene terephthalate polymerization, improving the dye affinity of the obtained fiber without damaging the color or thermal stability of the obtained polyester. The amount is preferably within a range from 0.0001 to 0.05 mg KOH/g, and more preferably from 0.0001 to 0.015 mg KOH/g.

The water used in condition (B) of the present invention can be any type capable of extracting the inorganic acid component constituting an impurity in the biomass resource-derived dicarboxylic acid and/or ester forming derivative thereof; examples include tap water, distilled water, ion-exchanged water, ultrapure water, and the like. For the sake of increased precision of analysis, distilled water, ion-exchanged water, or ultrapure water is preferable.

As described above, the biomass resource-derived dicarboxylic acid and/or ester forming derivative thereof according to the present invention includes an organic acid component and an inorganic acid component as acid components; as a rule, the organic acid component is extracted using an organic solvent, the inorganic acid component is extracted using water, and the amounts of potassium hydroxide necessary to neutralize these acid components differ. In other words, each of [these amounts] is an index indirectly and quantitatively expressing a different impurity content level. Specifically examples of organic acid component include a biomass resource-derived amino acid or a dicarboxylic acid monoalkyl ester constituting a substance resulting from incomplete reaction when a biomass resource-derived dicarboxylic acid dialkyl ester is used as a raw material. As a rule, such highly hydrophobic organic acids cannot be extracted by water, but can only be extracted by an organic solvent. Meanwhile, examples of inorganic acid component include biomass resource-derived inorganic acid components, along with inorganic acid components such as neutralizing agents, additives, catalysts, and the like used in the refining and synthesizing steps—for example, sulfuric acid, hydrobromic acid, hydrochloric acid, and the like—and, as a rule, such highly hydrophilic inorganic acids cannot be extracted by organic solvents, but can only be extracted by water.

In the present invention, it is preferable that (C) the sulfuric acid ion content of the biomass resource-derived dicarboxylic acid and/or ester forming derivative thereof be 40 ppm or less. If the sulfuric acid ion content is greater than 40 ppm, the color and thermal stability of the obtained polyester will be negatively affected. It is preferable that as small an amount as possible of sulfuric acid ions be used, but it is not realistic to reduce the amount to less than 0.01 ppm as this leads to excessive costs during the refining step; moreover, if small amounts of sulfuric acid ions remain present, there is a slight increase in the byproduction of diethylene glycol especially during polyethylene terephthalate polymerization, improving the dye affinity of the obtained fiber without damaging the color or thermal stability of the obtained polyester. The sulfuric acid ion content is preferably within a range from 0.01 to 40 ppm, and more preferably from 0.01 to 10 ppm.

It is preferable that the biomass resource-derived dicarboxylic acid and/or ester forming derivative thereof according to the present invention have an ash content of 100 ppm or less as determined by a method described hereafter, as this prevents coloration of the polyester and the generation of foreign particle. As small an ash content as possible is preferable, but it is not realistic to reduce the content to less than 0.01 ppm as this leads to excessive costs during the refining step. The ash content is preferably within a range from 0.01 to 100 ppm, more preferably from 0.1 to 80 ppm, and especially preferably from 0.5 to 50 ppm. In this specification, ash content is determined by precisely weighing the dicarboxylic acid and/or ester forming derivative thereof on a platinum dish (W1), combusting using an electric heater, further adding sulfuric acid and combusting, precisely weighing the residue left after performing ashing using an electric furnace at 550°C (W2), and calculating ash content according to the following formula.

$$\text{Ash content (ppm)} = (W2 / W1) \times 10^{6}$$

W1: Weight (g) of specimen precisely weighed on platinum dish
W2: Weight (g) of residue

Examples of the biomass resource-derived dicarboxylic acid and/or ester forming derivative thereof according to the present invention include aliphatic dicarboxylic acids such as succinic acid, adipic acid, azelaic acid, and sebacic acid and dicarboxylic acids and/or ester forming derivatives thereof such as terephthalic acid; of these, terephthalic acid and/or an ester forming derivative thereof is especially preferred. Especially preferable forms of terephthalic acid and/or ester forming derivative thereof include terephthalic acid dimethyl ester, terephthalic acid diethyl ester, terephthalic acid dipropyl ester, terephthalic acid dibutyl ester, terephthalic acid methyl(2-hydroxyethyl) ester, terephthalic acid di(2-hydroxyethyl) ester, and the like. These may be used singly or in combinations of two or more types. In particular, a terephthalic acid dimethyl ester is more preferably as it allows for distillation refining, enabling a high purity level to be obtained.

If the biomass resource-derived dicarboxylic acid and/or ester forming derivative thereof of the present invention is a dialkyl ester of a biomass resource-derived dicarboxylic acid, the dicarboxylic acid monoalkyl ester content is preferably 0.05% by weight or less. If the dicarboxylic acid monoalkyl ester content is greater than 0.05% by weight, the transesterification reactivity of the dialkyl ester of the dicarboxylic acid will be reduced.

There is no particular limitation upon the method used to obtain the biomass resource-derived dicarboxylic acid and/or ester forming derivative thereof according to the present invention, and any method may be used. Examples of methods of obtaining biomass resource-derived terephthalic acid include the method described in Patent Document 5 or PCT Publication WO/2010/148070. Other examples include the methods described in Patent Document 6, Unexamined Japanese Patent Application Publication 2007-176873, PCT Publication WO/2009/064515, PCT Publication WO/2009/079213, PCT Publication WO/2010/151346, Unexamined Japanese Patent Application Publication 2011-168501, and the like. Other examples include methods wherein the biomass resource-derived terephthalic acid is obtained by esterifying biomass resource-derived dicarboxylic acid using an alcohol such as methanol, ethanol, or the like. Of these, the method according to PCT Publication 2009/079213, wherein a biomass resource-derived paraxylene obtained by means of a process including the steps (1) through (3) described below is used to obtain a biomass resource-derived dicarboxylic acid and/or ester forming derivative thereof according to the process (4) described below, is especially preferable.

(1) Obtaining isobutanol from a biomass resource by means of a fermentation method
(2) Obtaining isobutene from the isobutanol by means of dehydration
(3) Converting the isobutene to paraxylene
(4) Obtaining a terephthalic acid and/or ester forming derivative thereof from the paraxylene

The diol component used in the method of producing a biomass resource-derived polyester according to the present invention is preferably a biomass resource-derived diol, as this allows the objectives of increasing the bio-based content and decreasing the amount of fossil resources used and increases in carbon dioxide to be achieved. The ratio of the biomass resource-derived diol to the total diol component content is preferably 80 mol% or more, and more preferably 100 mol%.

From considerations of obtaining a polyester with favorable physical properties, the diol is preferably at least one type selected from ethylene glycol, 1,3-propanediol, and 1,4-butanediol. Ethylene glycol is particularly preferable as it will raise the melting point of the obtained polymer.

There is no particular limitation upon the method used to obtain these diols from biomass resource, and any method may be used; examples include the methods described below.

One method of obtaining ethylene glycol from biomass resources is a method of obtaining ethylene glycol from biomass resources such as corn, sugar cane, wheat, or the stalks of agricultural crops. These biomass resources are first converted to starch, the starch is converted to glucose using water and enzymes, the glucose is converted to sorbitol by a hydrogenation reaction, the sorbitol becomes various glycol mixtures by means of a hydrogenation reaction at a fixed temperature and pressure in the presence of a catalyst, and the glycol mixtures are refined to obtain ethylene glycol. Another method is biologically processing a carbohydrate crop such as sugar cane or the like to obtain bioethanol, converting the bioethanol to ethylene, and passing through ethylene oxide to obtain ethylene glycol. Yet another method is obtaining glycerin from biomass resources, then passing through ethylene oxide to obtain ethylene glycol. The ethylene glycol so obtained contains various impurities; the level of impurities is preferably 1% by weight or less for each of 1,2-propanediol, 1,2-butanediol, 2,3-butanediol, and 1,4-butanediol; more preferably 0.5% by weight or less from considerations of the physical properties of the obtained polyester; and more preferably 0.1% by weight or less from considerations of the color of the obtained polyester.

There is no particular limitation upon the method used to obtain the 1,3-propanediol from the biomass resources; for example, it may be obtained by fermenting, then refining, a sugar such as glucose.

There is no particular limitation upon the method used to obtain the 1,4-butanediol from the biomass resources; for example, 1,4-butanediol can be obtained via chemical synthesis such as reduction or the like from succinic acid, succinic anhydride, succinic acid ester, maleic acid, maleic anhydride, maleic acid ester, tetrahydrofuran, gamma-butyrolactone, or the like obtained via fermentation.

In the production method according to the present invention, it is possible to include a copolymerizing component to the extent that the effects of the present invention are not substantially impeded. Examples of copolymerizing components include dicarboxylic acid components, including aromatic dicarboxylic acids such as isophthalic acid, 5-sulfoisophthalic acid salt, pthalic acid, naphthalene-2,6-dicarboxylic acid, biphenyl dicarboxylic acid, and the like and ester forming derivatives thereof, and aliphatic dicarboxylic acids such as succinic acid, adipic acid, pimelic acid, suberic acid, azelaic acid, sebacic acid, 1,9-nonane dicarboxylic acid, 1,12-dodecane dicarboxylic acid, and the like and ester forming derivatives thereof; and diol components such as propanediol, butanediol, pentanediol, hexanediol, polyethylene glycol with a molecular weight of from 500-20,000, diethylene glycol, 2-methyl-1,3-propanediol, polyoxytetramethylene glycol, polyoxytrimethylene glycol, bisphenol-A-ethylene oxide adduct, tetramethyl cyclobutanediol, and the like. These copolymerizing components can be used singly or in combinations of two or more types.

The most preferable aspect of the present invention is a method of producing a biomass resource-derived polyethylene terephthalate in which biomass resource-derived terephthalic acid and/or a biomass resource-derived terephthalic acid dimethyl ester is used as the dicarboxylic acid component and biomass resource-derived ethylene glycol is used as the diol component.

The biomass resource-derived polyester obtained using the present invention preferably has a bio-based content as determined by a method described below of 60% or more, as this will allow for further control of decreases in fossil resources and increases in carbon dioxide. In the context of present invention, " bio-based content " refers to the proportion of biomass resource-derived carbon as determined with reference to the concentration of radioactive carbon ($^{14}$ C) in circulation in the 1950s to the total amount of carbon atoms within the polymer as determined according to ASTM D6866. The bio-based content is preferably 70% or more, especially preferably 90% or more, and, if no copolymerizing component is used, most preferably 100%.

The method of producing a biomass resource-derived polyester according to the present invention is preferably a method in which an oligomer is obtained either by (1) esterifying a diol and a biomass resource-derived dicarboxylic acid or by (2) transesterifying a diol and an ester forming derivative of a biomass resource-derived dicarboxylic acid, performing a polymerization reaction under reduced interior reactor pressure, and obtaining a high molecular weight polyester.

In the method of producing a biomass resource-derived polyester according to the present invention, a compound of lithium, magnesium, manganese, calcium, cobalt, zinc, titanium, or the like may be used as a catalyst in the esterification reaction, or no catalyst may be used. The catalyst used in the transesterification reaction is, for example, a compound of lithium, magnesium, manganese, calcium, cobalt, zinc, titanium, or the like. The catalyst used in the polymerization reaction is, for example, a compound of antimony, titanium, aluminum, tin, germanium, or the like.

Examples of antimony compounds include antimony oxides, antimony carboxylate, antimony alkoxide, and the like; specific examples of antimony oxides including antimony trioxide and antimony pentoxide; specific examples of antimony carboxylates including antimony acetate, antimony oxalate, antimony potassium tartrate, and the like; and specific examples of antimony alkoxides including antimony tri-n-butoxide, antimony triethoxide, and the like.

Examples of titanium compounds include titanium titanium complexes, titanium alkoxides such as tetra-i-propyltitanate, tetra-n- butyltitanate, tetra-n- butyltitanate tetromers, and the like; titanium oxides obtained via the hydrolysis of titanium alkoxides, titanium acetyl acetonate, and the like. Of these, a titanium complex using a multivalent carboxylic acid and/or hydroxycarboxylic acid and/or polyhydroxy alcohol as a chelating agent is preferable from considerations of thermal stability and color of the polymer and reducing spinneret deposits. Examples of chelating agent used in the

titanium compound include lactic acid, citric acid, mannitol, tripentaerythritol, and the like.

Examples of aluminum compounds include aluminum carboxylates, aluminum alkoxides, aluminum chelate compounds, basic aluminum compounds, and the like; with specific examples including aluminum acetate, aluminum hydroxide, aluminum carbonate, aluminum ethoxide, aluminum isopropoxide, aluminum acetyl acetonate, basic aluminum acetate, and the like.

Examples of tin compounds include monobutyltin oxide, dibutyltin oxide, methylphenyltin oxide, tetraethyltin oxide, hexaethyltin oxide, triethyltin hydroxide, monobutylhydroxytin oxide, monobutyltin trichloride, dibutyltin sulfide, and the like.

Examples of germanium compounds include germanium oxides and germanium alkoxides; with specific examples of germanium oxides including germanium dioxide and germanium tetraoxide, and specific examples of germanium alkoxides including germanium tetraethoxide, germanium tetrabutoxide, and the like.

Specific examples of lithium compounds include lithium oxide, lithium hydroxide, lithium alkoxide, lithium acetate, lithium carbonate, and the like.

Specific examples of magnesium compounds include magnesium oxide, magnesium hydroxide, magnesium alkoxide, magnesium acetate, magnesium carbonate, and the like.

Specific examples of manganese compounds include manganese chloride, manganese bromide, manganese nitrate, manganese carbonate, manganese acetylacetonate, manganese acetate, and the like.

Specific examples of calcium compounds include calcium oxide, calcium hydroxide, calcium alkoxide, calcium acetate, calcium carbonate, and the like.

Specific examples of cobalt compounds include cobalt chloride, cobalt nitrate, cobalt carbonate, cobalt acetylacetonate, cobalt naphthenate, cobalt acetate tetrahydrate, and the like.

Specific examples of zinc compounds include zinc oxide, zinc alkoxide, zinc acetate, and the like.

In the method of producing a biomass resource-derived polyester according to the present invention, an antioxidant is preferably added. Because various trace impurities such as alkaline component derived from biomass resource-derived amino acids and proteins are present in the biomass resource-derived polyester, foreign particle may be formed under the high temperature conditions of the polymerization reaction, or coloration or worsening of the thermal stability of the polyester may occur. The addition of an antioxidant controls the formation of foreign particle and allows a biomass resource-derived polyester of superior thermal stability and polymer color to be obtained.

There is no particular limitation upon the antioxidant; examples include phosphorous-based, hindered phenol-based, sulfur-based, hydrazine-based, and triazole-based antioxidants. These may be used singly or in combinations of two or more types.

Examples of phosphorous-based antioxidants include phosphite compounds, phosphate compounds, phosphonite compounds, phosphonate compounds, phosphinite compounds, phosphinate compounds, and the like. Of these, phosphoric acid, trimethyl phosphate, triethyl phosphate, ethyl diethylphosphonoacetate, and the like are preferable as they demonstrate high foreign matter particle generation preventing effects and have good formability.

Phosphorous compounds such as bis(2,6-di-tert-butyl-4-methylphenyl)pentaerythritol-di-phosphite (PEP-36: Asahi Denka), represented by formula (1); tetrakis(2,4-di-t-butyl-5-methylphenyl) [1,1-biphenyl]-4,4'-diylbisphosphonate (GSY-P101: Osaki Industry), represented by formula (2); bis(2,4-di-tert-butylphenyl) pentaerythritol-di-phosphite (PEP-24G: Asahi Denka; IRGAFOS 126: Ciba Japan), represented by formula (3); and tetrakis(2,4-di-t-butylphenyl) [1,1-biphenyl]-4,4'-diylbisphosphonite (IRGAFOS P-EPQ: Ciba Japan; Sandostab P-EPQ: Clariant), represented by formula (4), are preferable from considerations of improved color and thermal stability. These phosphorous compounds may be used singly or in combinations of two or more types.

[Formula 1]

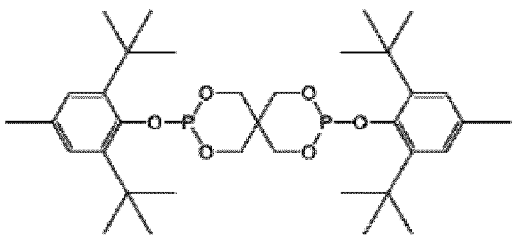

(1)

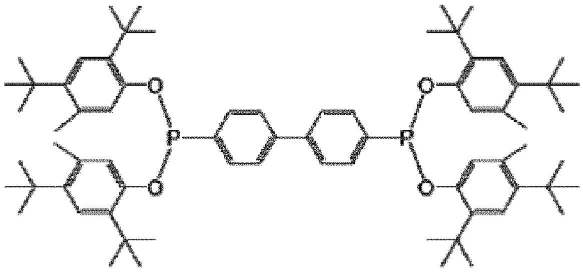

(2)

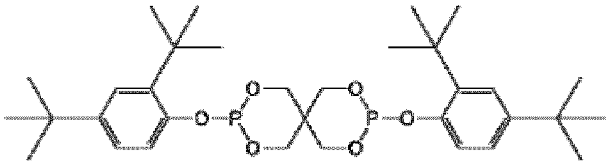

(3)

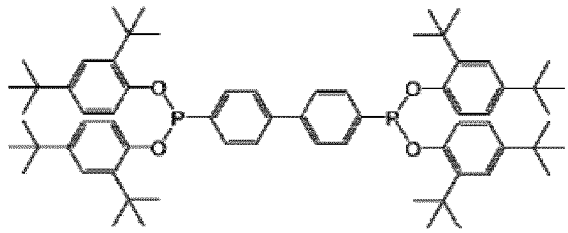

(4)

Examples of hindered phenol-based antioxidants include pentaerythritol tetrakis[3-(3,5-di-t-butyl-4-hydroxyphenyl) propionate], thiodiethylene bis[3-(3,5-di-t-butyl-4-hydroxyphenyl) propionate], octadecyl-3-(3,5-di-t-butyl-4-hydroxyphenyl)propionate, 4,6-bis(octylthiomethyl)-0-cresol, and the like. Of these, pentaerythritol tetrakis[3-(3,5-di-t-butyl-4-hydroxyphenyl) propionate] (IRGANOX 1010: Ciba Japan) is preferable because of its high coloration controlling effects.

Examples of sulfur-based antioxidants include dilauryl thiodipropionate, ditridecyl thiodipropionate, dimyristyl thiodipropionate, distearyl thiodipropionate, pentaerythritol-tetrakis(3-lauryl thiopropionate), pentaerythritol-tetrakis(3-dodecyl thiopropionate), and the like. Of these, pentaerythritol-tetrakis(3-lauryl thiopropionate) (Sumilize TP-D: Sumitomo Chemical) is preferable because of its high effectiveness in improving thermal stability and controlling coloration.

Examples of hydrazine-based antioxidants include decamethylene dicarboxylic acid-bis(N'-salicyloyl hydrazide), bis(2-phenoxypropionyl hydrazide) isophthalate, N-formyl-N'-salicyloyl hydrazine, and the like.

Examples of triazole-based antioxidants include benzotriazole, 3-(N-salicyloyl) amino-1,2,4-triazole, and the like.

There is no particular limit upon the amount of antioxidant added in the method of producing a biomass resource-derived polyester according to the present invention, but an amount within a range of 0.1 to 10,000 ppm with respect to the obtained polyester is preferable. Amounts within the above range control the formation of foreign particle, thus allowing a polyester with good formability, as well as superior color and thermal stability, to be obtained. The amount is more preferably in a range from 1 to 1,000 ppm, and especially preferably from 5 to 500 ppm.

The biomass resource-derived polyester obtained according to the present invention preferably has an intrinsicviscosity ($\eta$) as determined by a measuring method described below of from 0.4 to 2.0 $dlg^{-1}$. The intrinsic viscosity of the polyester referred to here is the value as measured at 25°C with ortho-chlorophenol as a solvent, and is an index expressing the mechanical properties of the polyester. If the intrinsic viscosity is less than 0.4 $dlg^{-1}$, mechanical properties will be insufficient, and it becomes difficult to obtain a molded product capable of withstanding applied use. If the polyester of the present invention is polyethylene terephthalate, an intrinsic viscosity of from 0.4 to 1.5 $dlg^{-1}$ is more preferable, and an intrinsic viscosity of from 0.6 to 1.3 $dlg^{-1}$ is especially preferable. If the polyester of the present invention is polypropylene terephthalate, an intrinsic viscosity of from 0.6 to 2.0 $dlg^{-1}$ is more preferable, and an intrinsic viscosity of from 0.7 to 1.6 $dlg^{-1}$ is especially preferable. If the polyester of the present invention is polybutylene terephthalate, an intrinsic viscosity of from 0.6 to 2.0 $dlg^{-1}$ is more preferable, and an intrinsic viscosity of from 0.8 to 1.8 $dlg^{-1}$ is especially preferable.

From considerations of color in molded products such as fibers and films, the color of the biomass resource-derived polyester obtained according to the present invention preferably has a Hunter b value in a range from -10 to 15 when in a pelletized chip form. The b value of the polyester is more preferably in a range from -5 to 15, still more preferably from -3 to 12, and especially preferably from 0 to 10. Hunter values (L, a, b) are measured according to the color system

described in JIS Z8730. The b value tends to increase the greater the organic acid component, inorganic acid component, or sulfuric acid ion content of the biomass resource-derived dicarboxylic acid and/or ester forming derivative thereof constituting the raw material is.

Furthermore, it is preferable that the color difference ΔE with a comparable fossil resource-derived polyester as determined by a method described hereafter be 1.5 or less, and a difference of 1.0 is more preferable as this effectively eliminates any distinctions visible to the eye. In this context, "comparable fossil resource-derived polyester" refers to a fossil resource-derived polyester formed from a fossil resource-derived dicarboxylic acid and a fossil resource-derived diol polymerized under the same conditions as for the biomass resource-derived polyester by performing esterification or transesterification under the same conditions.

A color adjusting agent may be used in the method of producing a biomass resource-derived polyester according to the present invention. As described above, the impurities contained in the raw materials of the biomass resource-derived polyester can cause coloration of the polyester, but using a color adjusting agent enables a good color to be obtained.

There is no particular limitation upon the color adjusting agent, which may be a dye or a pigment, but the use of a dye is preferable from consideration of formability. In particular, specific examples by Index Generic Name include blue color adjusting agents such as Solvent Blue 104,Solvent Blue 45, and the like; purple color adjusting agents such as Solvent Violet 36, Solvent Violet 8, and the like; red color adjusting agents such as Solvent Red 24, Solvent Red 25, and the like; and orange color adjusting agents such as Solvent Orange 60. Of these, blue color adjusting agents such as Solvent Blue 104 and Solvent Blue 45 and purple color adjusting agents such as Solvent Violet 36 and the like are preferable as they contain none of the halogens that often lead to apparatus corrosion, have comparatively good thermal stability at high temperatures, and demonstrate superior chromogenicity. These color adjusting agents may be used singly or in combinations of two types or more. There is no particular limitation upon the amount of color adjusting agent added, but an amount within a range of a total of 0.1 to 100 ppm with respect to the polyester is preferable as this yields a polyester with a high brightness. The amount is more preferably in a range from 0.5 to 20 ppm, and especially preferably from 1 to 5 ppm.

The biomass resource-derived polyester obtained according to the present invention preferably has a carboxyl terminal group content in a range from 1 to 50 equivalent /ton. The carboxyl terminal group content is preferably as low as possible, as this improves thermal stability, allows coloration of the poly mer when being remelted to be reduced, decreases deposits from forming around the spinneret during melt-spinning, and improves spinning stability. The carboxyl terminal group content is preferably 40 equivalent /ton or less.

The thermal stability of the biomass resource-derived polyester obtained according to the present invention is represented by the values "Δb 290" and "Δ carboxyl terminal group 290" as determined according to a method described below, with a Δb 290 of 2 or less and a Δ carboxyl terminal group 290 of 10 equivalent /ton or less being preferable. Δb 290 was found by vacuum drying the polyester at 150°C for twelve hours, heating and melting at 290°C for 60 minutes in a nitrogen atmosphere, measuring b according to the method described above, and taking the difference in b value before and after melting as Δb 290. Δ carboxyl terminal group 290 was found by vacuum drying the polyester at 150°C for twelve hours, heating and melting at 290°C for 60 minutes in a nitrogen atmosphere, measuring the carboxyl terminalgroup content, and taking the difference in carboxyl terminal group content before and after melting as Δ carboxyl terminal group 290.

The biomass resource-derived polyester obtained according to the present invention preferably has a diethylene glycol content within a range from 0.1 to 3.0% by weight, as this allow for favorable polyester thermal stability and polyester fiber chromogenicity. From considerations of polyester thermal stability, as little diethylene glycol as possible is preferable, but, from considerations of polyester fiber dye affinity, as much diethylene glycol as possible is preferable. Compared to polyesters obtained from fossil resource-derived polyester raw materials, polyesters obtained from biomass resource-derived polyester raw materials tend to have greater diethylene glycol contents; thus, biomass resource-derived polyester fibers have good dye affinity, but may have worsened thermal stability. For this reason, the use of a biomass resource-derived dicarboxylic acid and/or ester forming derivative thereof fulfilling at least one of the following conditions (A), (B), and (C) in production is necessary.

(A) The amount of potassium hydroxide needed to neutralize the acid component extracted using an organic solvent from 1 g of the dicarboxylic acid and/or ester forming derivative thereof is 0.1 mg KOH/g or less
(B) The amount of potassium hydroxide needed to neutralize the acid component extracted using water from 1 g of the dicarboxylic acid and/or ester forming derivative thereof is 0.05 mg KOH/g or less
(C) The sulfuric acid ion content is 40 ppm or less

The diethylene glycol content is preferably in a range from 0.3 to 3.0% by weight, and especially preferably within a range from 1.0 to 2.5% by weight, as this allows for dye affinity to be improved while also yielding color and thermal stability comparable to those of fossil resource-derived products.

UV absorbers, flame retardants, fluorescent whitening agents, delustrants, plasticizers, anti-foaming agents, or other additives may also be included in the method of producing a biomass resource-derived polyester according to the present invention as necessary. In particular, when the biomass resource-derived polyester is used to make fibers, it is preferable to add titanium oxide as this can impart a brightness and opacity favorable for fibers used in clothing.

In the method of producing a biomass resource-derived polyester according to the present invention, solid phase polymerization may be performed in order to obtain a more highly polymeric polyester. There is no particular limitation upon the apparatus and method used for the solid phase polymerization, but it is performed by means of heating in an inert gaseous atmosphere or in a vacuum. Any inert gas that is inert with respect to polyester is acceptable, with possible examples including nitrogen gas, helium gas, carbon dioxide gas, or the like; but, from economic considerations, nitrogen is preferable. If the polymerization is performed in a vacuum, the pressure within the apparatus is preferably 133 Pa or less, and increasing the degree of vacuum is advantageous as this allows the time necessary for the solid phase polymerization reaction to be shortened.

The biomass resource-derived polyester obtained according to the present invention can also be recycled. Specifically, discarded biomass resource-derived polyester can be used as raw material for depolymerization using a biomass resource-derived or fossil resource-derived diol component to obtain bis(hydroxyalkyl)terephthalate. The bis(hydroxyalkyl)terephthalate may be repolymerized, but is preferably transesterified using methanol, ethanol, or the like into terephthalic acid dimethyl ester or terephthalic acid diethyl ester, enabling refinement to a high degree of purity by means of distillation. When repolymerization using this terephthalic acid dialkyl ester is performed, polymerization reactivity is good, and a polyester having little coloration can be obtained.

The method of producing a biomass resource-derived polyester according to the present invention can use any of batch polymerization, semicontinuous polymerization, or continuous polymerization.

The polyester produced by the method of producing a biomass resource-derived polyester according to the present invention can be process using normal polyester processing methods, is suited for use in molded products such as fibers, films, sheets, bottles, and resins, and can be manufactured into various types of end products.

Fibers formed using the biomass resource-derived polyester obtained according to the present invention can be used for clothing, industrial material, or medical uses; and the polyester is favorably used for clothing, in which a high degree of design freedom is demanded, because of its low level of coloration, and for industrial materials because of its high thermal stability. A favorable industrial materials use is as fibers for use in automobiles; for example, interior materials such as car seats, seatbelts, and ceiling materials and rubber reinforcing fibers for tires. In particular, because carbon dioxide increases can be suppressed even when the biomass resource-derived polyester of the present invention is burned, the polyester is favorably used as tire rubber reinforcing fibers, such as in carcasses or cap plies, which are often thermally recycled by combustion after use and for which a high degree of thermal stability is demanded.

Normal processes of melt-spinning and drawing may be applied as methods of obtaining fibers from the biomass resource-derived polyester of the present invention. It is possible to obtain undrawn thread by heating polyester pellets to melting point or above and melting the pellets, expelling the molten polyester through fine holes, cooling and solidifying the polyester cold air, applying an oil, pulling the solidified polyester using a pulling roller, and taking up the polyester using a takeup apparatus disposed after the pulling roller. The undrawn thread taken up in this way is drawn by one or more pairs of heated rollers and finally heat-treated in a tensed or slackened state to create polyester fibers imparted with dynamic characteristics and other physical properties according to the intended use. The drawing step can be performed immediately after pulling the thread in the melt-spinning step described above without first taking the thread up, and it is preferable from considerations of productivity and other industrial concerns that such continuous drawing be performed. When applying this drawing-heating treatment, the draw ratio, drawing temperature, and heat treatment conditions can be selected to suit the desired fiber fineness, strength, elongation, contraction rate, and the like.

The fibers obtained using the biomass resource-derived polyester obtained according to the present invention preferably have a dye uptake rate of 90% or more. In this context, "dye uptake rate" is a value obtained by using a spectrophotometer (Hitachi U-3000) to measure the absorbency of a dyebath before and after dyeing is performed under the conditions described below and calculated using the following formula, and acts as an index expressing the dye affinity and chromogenicity of the polyester fibers. Dyeing conditions were as follows.

- Dye: Sumikaron Blue S-3RF (A) (azo disperse dye, Sumitomo Chemtex)
- Concentration: 3% owf
- Bath ratio: 1:20
- Dyeing temperature: 110°C
- Dyeing time: 60 minutes

$$\text{Dye uptake rate (\%)} = ((\text{Abs0}-\text{Abs1})/\text{Abs0}) \times 100$$

(Abs0 is absorbency at the maximum light absorption wavelength of the dyebath prior to dyeing; Abs1 is absorbency at the maximum light absorption wavelength of the dyebath after dyeing)

A dye uptake rate of 95% or more leads to good chromogenicity and hyperchromicity, yielding fibers with superior dye affinity. A dye uptake rate of 90% or more is more preferable, and a dye uptake rate of 95% or more is especially preferable. Examples

The present invention will be explained in greater detail below with the aid of examples. Physical property values of the examples were measured according to the following methods.

(1) Amount of potassium hydroxide needed to neutralize the acid component extracted using an organic solvent from the dicarboxylic acid and/or ester forming derivative thereof (unit: mg KOH/g)

1 g of a dicarboxylic acid and/or ester forming derivative thereof was dissolved in a titration solvent consisting of a 1:2 mixture of ethanol and paraxylene, and using phenol red and bromothymol blue as indicators, titration was performed using a 0.1 mol/L potassium hydroxide/ethanol solution to find the amount.

(2) Amount of potassium hydroxide needed to neutralize the acid component extracted using water from the dicarboxylic acid and/or ester forming derivative thereof (unit: mg KOH/g)

1 g of dicarboxylic acid and/or ester forming derivative thereof was added to 30 mL ion-exchanged water and stirred for 30 minutes. After stirring, titration was performed using a 0.1 mol/L potassium hydroxide/ethanol solution to find the amount, using phenol red and bromothymol blue as indicators.

(3) Sulfuric acid ion content of the dicarboxylic acid and/or ester forming derivative thereof (unit: ppm)

0.2 g of dicarboxylic acid and/or ester forming derivative thereof was added to 15 mL ion-exchanged water and stirred for 30 minutes. After stirring, the dicarboxylic acid and/or ester forming derivative thereof was filtered out using a syringe filter. The filtrate was subjected to ion chromatography to quantify and find the sulfuric acid ion ($SO_4^{2-}$) content.

<Ion chromatography conditions>

- Column: TSK-GEL SUPER IC-AZ (4.6 mm I.D. x 15 cm)
- Eluent: 2.9 mmol/L $NaHCO_3$ / 3.1 mmol/L $Na_2CO_3$
- Detector: Conductance meter

(4) Ash content of the dicarboxylic acid and/or ester forming derivative thereof (unit: ppm)

Ash content was determined by precisely weighing the dicarboxylic acid and/or ester forming derivative thereof on a platinum dish, combusting using an electric heater, further adding sulfuric acid and combusting, precisely weighing the residue left after performing ashing using an electric furnace at 550°C, and calculating ash content according to the following formula.

$$\text{Ash content (ppm)} = (W2 / W1) \times 10^6$$

W1: Weight (g) of specimen precisely weighed on platinum dish
W2: Weight (g) of residue

(5) Measuring the monoalkyl ester content of the dicarboxylic acid and/or ester forming derivative thereof (unit: weight%)

0.05 g dicarboxylic acid and/or ester forming derivative thereof was precisely weighed in a 100 mL volumetric flask, and acetonitrile was added to the benchmark. After the dicarboxylic acid and/or ester forming derivative thereof was completely dissolved, HPLC analysis was performed.

<HPLC analysis conditions>

- Column: Inertsil ODS-3 (4.6 mm ID x 250 mm, df = 5 μm)
- Eluent: (liquid A) 0.1 % aqueous phosphoric acid solution, (liquid B) acetonitrile

| Time | 0 min | 20 min | 25 min | 30 min |
|---|---|---|---|---|
| B concentration | 30 | 70 | 30 | 30 |

- Flow rate: 1.0 mL/min
- Column temperature: 40°C
- Detection wavelength: 240 nm
- Injection volume: 2 μl

(6) Polyester intrinsic viscosity ([η], units $dlg^{-1}$)

Measuring was performed using ortho-chlorophenol at 25°C.

(7) Polymer melting point (Tm, unit: °C)

First, using a DSC apparatus, the temperature was raised from 40°C to 280°C at a rate of 16°C/min and maintained at that level for three minutes, the heat history was removed, and the temperature was then reduced to 40°C at a rate of 16°C/min and maintained at that level for three minutes. Finally, the temperature was raised to 280°C at a rate of 16°C/min, and the melting temperature obtained during the second heating process was taken as the melting point (Tm).

(8) Polymer color

Using a color difference meter (Suga Test Instruments, SM color computer model SM-T45), the color of the polymer was measured in terms of Hunter values (L, a, b values) according to the color system described in JIS Z8730.

(9) Color difference (ΔE)

Color difference (ΔE) with a corresponding fossil resource-derived polyester was calculated according to the following formula, with the color of the biomass resource-derived polyester expressed as values (Lb, ab, bb), and the color of the corresponding fossil resource-derived polyester expressed as values (Lp, ap, bp).

$$\Delta E = ((Lb - Lp)2 + (ab - ap)2 + (bb-bp)2)1/2$$

(10) Polymer carboxyl terminal group content (unit: equivalent / ton)

With ortho-cresol as a solvent, a 0.02 standard NaOH aqueous solution was used to perform titration at 25°C using an automated titration apparatus (Hiranuma Sangyo COM-550) and measure the content.

(11) Polymer thermal stability index (Δb 290, Δ carboxyl terminal group 290)

The index was found by vacuum drying the polyester at 150°C for twelve hours, heating and melting at 290°C for 60 minutes in a nitrogen atmosphere, measuring the color and carboxyl terminal group content according to the method described above, and finding Δb 290 and Δ carboxyl terminal group 290 as the differences before and after heating and melting, respectively.

(12) Polymer diethylene glycol (DEG) content (unit: weight%)

A sample was heated in the presence of 1,6-hexanediol using monoethanolamine as a solvent, cooled after methanol was added thereto, and neutralized with acid, and the diethylene glycol content was measured by centrifuging off the supernatant and performing gas chromatography (Shimadzu Corp. GC-14A).

(13) Polymer phosphorus content (unit: ppm)

A sample in chip form was heated and melted upon an aluminum plate, a molded piece having a flat surface was formed using a compression press, and phosphorus content was determined using an x-ray fluorescence elemental analyzer (Rigaku Corp. System 3270).

(14) Polymer elemental sulfur content (unit: ppm)

A sulfuric acid ion standard solution (1004 mg/L, Wako Pure Chemical Industries Lot. DCQ4971) was progressively diluted using a separately prepared phosphoric acid internal standard solution to prepare a standard solution. Of these, analysis data for a standard solution suitable for analyzing the concentration in the specimen was used to create a calibration curve.

Meanwhile, approximately 0.1 g of the specimen was weighed on a platinum boat and combusted within the combustion tube of the analytical apparatus described below, the gas generated was absorbed using a solution, and a part of the absorption liquid was analyzed using ion chromatography.

Analysis apparatus: AQF-100, GA-100 (Mitsubishi Chemical)
Electric furnace temperature: inlet: 900°C; outlet: 1,000°C
Gas: $Ar/O_2$ 200 mL/min
$O_2$ 400 mL/min
Absorption liquid: $H_2O_2$ 0.1%, internal standard P1 μg/mL
Absorption liquid amount: 10 mL

(15) Method of measuring bio-based content

The bio-based content was determined according to ASTM D6866. Specifically, a sample was pulverized using sandpaper and a pulverizer, heated along with copper oxide, and completely oxidized into carbon dioxide, which was reduced to graphite using iron powder in order to effect conversion to a single-carbon compound. The obtained graphite sample was introduced into an AMS apparatus and the $^{14}C$ concentration was measured. The $^{14}C$ concentration of the standard substance oxalic acid (supplied by the U.S. National Institute of Standards and Technology) was simultaneously measured. $\Delta^{14}C$ was determined according to the following formula, wherein $^{14}As$ is the ratio of carbon-14 to carbon-12 ($^{14}C/^{12}C$) in the sample, and $^{14}Ar$ is the ratio of carbon-14 to carbon-12 ($^{14}C/^{12}C$) in the standard substance.

$$\Delta^{14}C = ((^{14}As - ^{14}Ar)/^{14}Ar) \times 1000$$

The $\Delta^{14}C$ was used to determine the percent modem carbon (pMC) according to the following formula.

$$pMC = \Delta^{14}C/10 + 100$$

The pMC was multiplied by 0.93 (=100/107.5) as shown in the following formula in order to determine the bio-based content according to American Society for Testing and Materials (ASTM) standard D6866.

$$\text{Bio-based content (\%)} = 0.93 \times pMC$$

(16) Fiber dye uptake rate

Dye uptake rate was obtained by using a spectrophotometer (Hitachi U-3000) to measure the absorbency of a dyebath before and after dyeing and calculating using the following formula.

$$\text{Dye uptake rate (\%)} = ((Abs0-Abs1)/Abs0) \times 100$$

(Abs0 is absorbency at the maximum light absorption wavelength of the dyebath prior to dyeing; Abs1 is absorbency at the maximum light absorption wavelength of the dyebath after dyeing)

The materials used in the examples were as follows.

Production example 1 <Isobutanol fermentation>

Using microorganisms derived from freezer stock (for example, *Escherichia coli* modified to produce isobutanol) in 40 mL of a modified M9 medium consisting of glucose 85 g/L, yeast extract 20 g/L, ferric citrate 20 $\mu$M, $H_3BO_3$ 5.72 mg/L, $MnCl_2•4H_2O$ 3.62 mg/L, $ZnSO_4•7H_2O$ 0.444 mg/L, $Na_2MnO_4•H_2O$ 0.78 mg/L, $CuSO_4•5H_2O$ 0.158 mg/L, $CoCl_2•6H_2O$ 0.0988 mg/L, $NaHPO_4$ 6.0 g/L, $KH_2PO_4$ 3.0 g/L, NaCl 0.5 g/L, $NH_4Cl$ 2.0 g/L, $MgSO_4$ 0.0444 g/L, and $CaCl_2$ 0.00481 g/L, a culture was started overnight with a culture $OD_{600}$ of from 0.02 to 0.05 in a 250 mL Erlenmeyer flask. The starter culture was grown for approximately 14 hours at 30°C and 250 rpm in a shaker apparatus. Next, part of the starter culture was transferred to a 400 mL DASGIP fermentation apparatus containing approximately 200 mL modified M9 medium and an initial culture $OD_{600}$ of approximately 0.1 was achieved. The container was installed in a computer control system, and fermentation at pH 6.5 (obtained by adding alkali as necessary, the temperature of 30°C, and dissolved oxygen levels and stirring were monitored and controlled. The container was stirred at a minimum stirring speed of 200 rpm. Stirring was varied by using a 12 sl/hour air jet to maintain a dissolved oxygen content of approximately 50% saturation until $OD_{600}$ reached approximately 1.0. Afterwards, the container was induced using 0.1 mM 1PTG. After approximately 8 to 10 hours of continuous growth, dissolved oxygen content was reduced to 5% at the minimum stirring speed of 200 rpm and 2.5 sl/hour air current. Throughout the entire experiment, continuous measurement of fermentation tank container off-gas by means of GC-MS analysis was performed for oxygen, isobutanol, ethanol, carbon dioxide, and nitrogen. Throughout the entire fermentation process, specimens were aseptically removed from the fermentation tank container and were used to measure the $OD_{600}$, glucose concentration, and isobutanol concentration of the broth. Isobutanol levels reached maximum around 21.5 hours, titer was 18 g/L, and yield was approximately 70% of the maximum theoretical value. The broth was subjected to vacuum distillation, an 84:16 mixture of isobutanol and water was provided, and the mixture was redistilled as necessary to obtain dry isobutanol.

Production example 2 <Isobutanol-derived diisobutylene>

The isobutanol produced by fermentation according to production example 1 was separated from the fermentation broth by distillation. Isobutanol containing 16% water was introduced at approximately 10 psig and an WHSV of 6 $hr^{-1}$ into a chemical reactor apparatus containing a commercially available gamma-alumina catalyst heated to 310°C. Water drained from the bottom of the reactor apparatus contained less than 0.1 M isobutanol, and isobutylene (gaseous) was gathered at a conversion rate of greater than 99%. The isobutylene gas was dried by being passed through a molecular sieve and supplied to a second reactor apparatus containing ZSM-5 catalyst maintained at from 140 to 160°C, atmospheric pressure, and WHSV 1.5 $hr^{-1}$, and a mixture of approximately 80% diisobutylene isomer, approximately 20% triisobutylene isomer, and trace amounts of higher polymer products was obtained at a conversion rate of approximately 60%.

Production example 3 <Diisobutylene-derived xylene>

Diisobutylene prepared from isobutanol as described in production example 2 was supplied to a reactor apparatus contained a chromium-doped eta-alumina catalyst. The reactor apparatus was maintained at a WHSV of 1.1 $hr^{-1}$ and 550°C. The reaction product was condensed and analyzed by GC-MS. The xylene fraction yield was approximately 20%, and p-xylene was obtained at approximately 90% selectivity.

The biomass paraxylene synthesized according to production examples 1 through 3 was produced according to PCT Publication WO/2009/079213 as described above, and is equivalent to biomass paraxylene produced by Gevo.

Production example 4 <Biomass resource-derived terephthalic acid>

600 g acetic acid (acetic acid : water = 90:10), 0.76 g cobalt (III) acetate tetrahydrate, 0.60 g manganese (II) acetate tetrahydrate, and 0.66 mL hydrobromic acid (hydrogen bromide content 47 wt%) were introduced to a 1 L autoclave and pressurized to 0.8 MPa with oxygen-nitrogen gas (oxygen content 10 wt%) and heating was performed in an oil bath while stirring at 1,000 rpm. When internal temperature reached 180°C, continuous supply of the p-xylene obtained in production example 3 to the autoclave at a rate of 0.53 mL/min. Concurrently with the beginning of the paraxylene supply, internal pressure was adjusted to 1.6 MPa using air, air was continuously supplied so as to yield an exhaust gas flow of 2.2 mL/min, and an air oxidation reaction of the paraxylene was begun. While performing adjustment so that the internal temperature during the reaction was 190$\pm$5°C, the reaction was performed for three hours, the reaction product was filtered off and cleansed using acetic acid, and a biomass resource-derived terephthalic acid with a 4-carboxybenzaldehyde (4-CBA) concentration of 300 ppm or less was obtained at a yield of 98 mol% or more.

Comparative example 1-1 <Biomass resource-derived dimethyl terephthalate>

210 g of the biomass resource-derived terephthalic acid obtained in production example 4, 400 g methanol, 30 mL 98 wt% sulfuric acid, and 5.5g copper (II) sulfate pentahydrate were introduced to a 1 L autoclave, and an esterification reaction was performed at 110°C and 1.5 MPa. The reaction product was filtered off, and esterification was again performed under identical conditions. The reaction product was then filtered off and repeatedly cleansed using methanol to obtain dimethyl terephthalate at a yield of 86 mol%. Hereafter, the biomass resource-derived dimethyl terephthalate may be referred to as "DMT-b".

Example 1-1 <Distillation of biomass resource-derived dimethyl terephthalate under alkaline conditions>

0.018 parts by weight of sodium carbonate were added to 100 parts by weight of the dimethyl terephthalate obtained in comparative example 1-1 and simple distillation was performed at 185°C and 56 hPa to obtain a biomass resource-derived dimethyl terephthalate. Recovery rate was 95%. The 0.018 parts by weight of sodium carbonate used here is equivalent to equal to or greater than the amount of potassium hydroxide (0.19 mg KOG/g) necessary to neutralize the acid component of the dimethyl terephthalate obtained in comparative example 1-1 as described in (2) above and equal to or greater than the amount of sulfuric acid ions (138 ppm) determined as measured according to (3) above.

Example 1-2 <Simple distillation of biomass resource-derived dimethyl terephthalate>

Simple distillation of the dimethyl terephthalate obtained in comparative example 1-1 was performed at 185°C and 56 hPa to obtain a biomass resource-derived dimethyl terephthalate. Recovery rate was 80%.

Example 1-3 <Simple distillation of biomass resource-derived dimethyl terephthalate>

Simple distillation of the dimethyl terephthalate obtained in comparative example 1-1 was performed at 185°C and 56 hPa to obtain a biomass resource-derived dimethyl terephthalate. Recovery rate was 85%.

Example 1-4 <Simple distillation of biomass resource-derived dimethyl terephthalate>

Simple distillation of the dimethyl terephthalate obtained in comparative example 1-1 was performed at 185°C and 56 hPa to obtain a biomass resource-derived dimethyl terephthalate. Recovery rate was 90%.

Example 1-5 <Simple distillation of biomass resource-derived dimethyl terephthalate>

Simple distillation of the dimethyl terephthalate obtained in comparative example 1-1 was performed at 185°C and 56 hPa to obtain a biomass resource-derived dimethyl terephthalate. Recovery rate was 95%.

Example 1-6 <Simple distillation of biomass resource-derived dimethyl terephthalate>

Simple distillation of the dimethyl terephthalate obtained in comparative example 1-1 was performed at 185°C and 56 hPa to obtain a biomass resource-derived dimethyl terephthalate. Recovery rate was 99%.

<Fossil resource-derived dimethyl terephthalate>

Comparative example 1-2

Dimethyl terephthalate produced by SK Chemicals was used as a fossil resource-derived dimethyl terephthalate. Hereafter, the fossil resource-derived dimethyl terephthalate may be referred to as "DMT-fossil".

Table 1 summarizes the results for (1) through (5) described above—namely, (1) the amount of potassium hydroxide necessary to neutralize the acid component extracted using an organic solvent, (2) the amount of potassium hydroxide necessary to neutralize the acid component extracted using water, (3) sulfuric acid ion content, (4) ash content, and (5) monoalkyl ester content—measured for the dimethyl terephthalates of the above Example 1-1 through example 1-6 and comparative examples 1-1 and 1-2

[Table 1]

| | Raw material X | Amt. of KOH needed to neutralize acid component extracted from raw material X using organic solvent (mg KOH/g) | Amt. of KOH needed to neutralize acid component extracted from raw material X using water (mg KOH/g) | Sulfuric acid ion content (ppm) | Ash content (ppm) | Monoalkyl ester content (ppm) |
|---|---|---|---|---|---|---|
| Example 1-1 | DMT-b | 0.001 | 0.001 | 0.1 | 0.1 | 0.01 |
| Example 1-2 | DMT-b | 0.005 | 0.013 | 10 | 1 | <0.01 |
| Example 1-3 | DMT-b | 0.004 | 0.049 | 35 | 1 | <0.01 |
| Example 1-4 | DMT-b | 0.006 | 0.072 | 48 | 1 | 0.04 |
| Example 1-5 | DMT-b | 0.02 | 0.15 | 98 | 5 | 0.06 |
| Example 1-6 | DMT-b | 0.08 | 0.15 | 99 | 61 | 0.06 |
| Comparative example 1-1 | DMT-b | 0.18 | 0.19 | 138 | 104 | - |
| Comparative example 1-2 | DMT-fossil | 0.005 | 0 | Undetectable | 3 | <0.01 |

Abbreviations used in Table 1 are as follows.

- DMT-b: biomass resource-derived dimethyl terephthalate
- DMT-fossil: fossil resource-derived dimethyl terephthalate

<Biomass resource-derived ethylene glycol>

Ethylene glycol produced by India Glycols was used. Hereafter, biomass resource-derived ethylene glycol may be referred to as "EG-b".

<Fossil resource-derived ethylene glycol>

Ethylene glycol produced by Nippon Shokubai was used. Hereafter, the fossil resource-derived ethylene glycol may be referred to as "EG-fossil".

<Production and evaluation of polyethylene terephthalate>

Example 2-1

100 parts by weight of the biomass resource-derived dimethyl terephthalate ("DMT-b" in the table) obtained in example 1-2, 60 parts by weight of biomass resource-derived ethylene glycol (India Glycols), and an amount of magnesium acetate ("MGA" in the table) equivalent to 620 ppm with respect to the obtained polymer were melted in a transesterification reactor at 150°C in a nitrogen atmosphere and heated over four hours to 240°C while stirring, methanol was distilled out, a transesterification reaction was performed, and bis(hydroxyethyl) terephthalate was obtained. The bis(hydroxyethyl) terephthalate was transferred to a polycondensation tank.

After being transferred, the bis(hydroxyethyl) terephthalate was pre-mixed in biomass resource-derived ethylene glycol (India Glycols) in a separate mixing tank 30 minutes before the equivalent of 300 ppm of antimony trioxide ("$Sb_2O_3$" in the table) with respect to the obtained polymer and 200 ppm of trimethyl phosphate ("TMPA" in the table) with respect to the obtained polymer were added, and the mixture was stirred for 30 minutes at room temperature before being added thereto. Five minutes later, the reaction system was depressurized and reaction begun. The temperature within the reactor was gradually increased from 250°C to 290°C and pressure was reduced to 40 Pa. The amount of time before the final temperature and final pressure were reached was 60 minutes in both cases. After a predetermined stirring torque was reached, the reaction system was purged of nitrogen and returned to normal pressure, the polycondensation

reaction was stopped, and strands were expelled, cooled, and immediately cut to obtain polyethylene terephthalate pellets. The amount of time from the start of depressurization until the predetermined stirring torque was reached was two hours and twenty minutes, and polymerization reactivity was good.

The obtained polyethylene terephthalate had both good color and good thermal stability, and had polymer properties in no way inferior to those of a fossil resource-derived polyester produced under identical conditions (comparative example 2-4).

Polymer properties are summarized in Table 3.

The polyethylene terephthalate was then spun and dyed in a manner identical to that of example 2-13, described below. When the obtained fibers were evaluated, the dye uptake rate was 95%.

Example 2-2

Polyethylene terephthalate was obtained in the same manner as in example 2-1, except that the biomass resource-derived dimethyl terephthalate obtained in example 1-6 was used. Polymerization time and polymer properties are summarized in Table 3. As the amount of potassium hydroxide needed to neutralize the acid component extracted from the biomass resource-derived dimethyl terephthalate using an organic solvent or water, sulfuric acid ion content, and ash content increased, lengthened polymerization reaction time, polymer color discoloration, and worsened thermal stability were observed, and somewhat large lengthening of the polymerization reaction time and discoloration of the polymer were observed.

Example 2-3

Polyethylene terephthalate was obtained in a manner identical to that of example 2-1, except that an amount of a titanium citrate complex chelate equivalent to 15 ppm titanium atoms with respect to the obtained polymer was used instead of the antimony trioxide as a polymerization catalyst. Polymerization time and polymer properties are summarized in Table 3. Polymerization reactivity was good, and the obtained polyethylene terephthalate had both good color and good thermal stability.

Example 2-4

Polyethylene terephthalate was obtained in a manner identical to example 2-1, except that the biomass resource-derived ethylene glycol was changed to a fossil resource-derived ethylene glycol (Nippon Shokubai; "EG-fossil" in the table). Polymerization time and polyethylene terephthalate properties are summarized in Table 3. Polymerization reactivity was good, and the obtained polyethylene terephthalate had both good color and good thermal stability, but the bio-based content decreased.

Examples 2-5 through 2-8

Polyethylene terephthalate was obtained in a manner identical to that of example 2-1, except that the antioxidant phosphorus compound (TMPA) was changed to phosphoric acid ("PA" in the table), the phosphorus compound bis(2,6-di-tert-butyl-4-methylphenyl) pentaerythritol-di-phosphite (Asahi Denka; "PEP-36" in the table), the phosphorus compound tetrakis(2,4-di-t-butyl-5-methylphenyl) [1,1-biphenyl]-4,4'-diylbisphosphonite (Osaki Industry; "GSY" in the table), and the hindered phenol compound pentaerythritol tetrakis[3-(3,5-di-t-butyl-4-hydroxyphenyl) propionate] (Ciba Japan; "IR1010" in the table), as shown in Table 2. Polymerization time and polyethylene terephthalate properties are summarized in Table 3.

Polymerization reactivity was good, and the obtained polyethylene terephthalate had both good color and good thermal stability.

Examples 2-9 and 2-10

Polyethylene terephthalate was obtained in a manner identical to example 2-1, except that that amount of phosphorus compound (TMPA) added was changed as shown in Table 2. Polymerization time and polyethylene terephthalate properties are summarized in Table 3. Example 2-9, which had a low amount of added phosphorus compound, showed somewhat poor thermal stability.

Example 2-11

Polyethylene terephthalate was obtained in a manner identical to example 2-1, except that 2 ppm of color

adjusting agent with respect to the polymer obtained as a biomass resource-derived ethylene glycol (India Glycols) solution was added during polymerization. Polymerization time and polyethylene terephthalate properties are summarized in Table 3. Polymerization reactivity was good.

Example 2-12

Polyethylene terephthalate was obtained in a manner identical to example 2-1, except that 0.3% by weight as calculated in titanium oxide particles of a titanium oxide particle biomass resource-derived ethylene glycol (India Glycols) slurry was added to the obtained polymer during polymerization. Polymerization time and polyethylene terephthalate properties are summarized in Table 3. Polymerization reactivity was good, and the obtained polyethylene terephthalate had both good color and good thermal stability.

Comparative example 2-1

Polyethylene terephthalate was obtained in the same manner as in example 2-1, except that the dimethyl terephthalate was changed to the biomass resource-derived dimethyl terephthalate synthesized in comparative example 1-1. When the biomass resource-derived dimethyl terephthalate, which required a large amount of potassium hydroxide in order to neutralize the acid component extracted by the organic solvent, was used, the polycondensation reaction did not reach the targeted torque, and a polyethylene terephthalate of low viscosity could not be obtained. Moreover, the polymer color showed a dramatic yellow discoloration, and thermal stability was also poor. Polyethylene terephthalate properties are summarized in Table 3.

[Table 2]

| | Material | | | Catalyst | | | | Additive | | | | |
|---|---|---|---|---|---|---|---|---|---|---|---|---|
| | Dicarboxylic acid component | | Diol component | Transesterification catalyst | | Polymerization catalyst | | Antioxidant | | Color adjusting agent | | $TiO_2$ |
| | | | | Type | Amount added (ppm) | Type | Amount added (ppm) | Type | Amount added (ppm) | Type | Amount added (ppm) | Amount added (wt%) |
| Example 2-1 | DMT-b | Example 1-2 | EG-b | MGA | 620 | $Sb_2O_3$ | 300 | TMPA | 200 | - | - | - |
| Example 2-2 | DMT-b | Example 1-6 | EG-b | MGA | 620 | $Sb_2O_3$ | 300 | TMPA | 200 | - | - | - |
| Example 2-3 | DMT-b | Example 1-2 | EG-b | MGA | 620 | Ti citrate | 15 (Ti atoms) | TMPA | 200 | - | - | - |
| Example 2-4 | DMT-b | Example 1-2 | EG-fossil | MGA | 620 | $Sb_{2O}3$ | 300 | TMPA | 200 | - | - | - |
| Example 2-5 | DMT-b | Example 1-2 | EG-b | MGA | 620 | $Sb_2O_3$ | 300 | PA | 200 | - | - | - |
| Example 2-6 | DMT-b | Example 1-2 | EG-b | MGA | 620 | $Sb_2O_3$ | 300 | PEP-36 | 200 | - | - | - |
| Example 2-7 | DMT-b | Example 1-2 | EG-b | MGA | 620 | $Sb_2O_3$ | 300 | GSY | 200 | - | - | - |
| Example 2-8 | DMT-b | Example 1-2 | EG-b | MGA | 620 | $Sb_2O_3$ | 300 | IR1010 | 1000 | - | - | - |
| Example 2-9 | DMT-b | Example 1-2 | EG-b | MGA | 620 | $Sb_2O_3$ | 300 | TMPA | 20 | - | - | - |
| Example 2-10 | DMT-b | Example 1-2 | EG-b | MGA | 620 | $Sb_2O_3$ | 300 | TMPA | 1000 | - | - | - |
| Example 2-11 | DMT-b | Example 1-2 | EG-b | MGA | 620 | $Sb_2O_3$ | 300 | TMFA | 200 | Blue 45 | 2 | - |
| Example 2-12 | DMT-b | Example 1-2 | EG-b | MGA | 620 | $Sb_2O_3$ | 300 | TMPA | 200 | - | - | 0.3 |
| Comparative example 2-1 | D/T-b | Comparative example 1-1 | EG-b | MGA | 620 | $Sb_2O_3$ | 300 | TMPA | 200 | - | - | - |

Abbreviations used in Table 2 are as follows.

- DMT-b: biomass resource-derived dimethyl terephthalate
- EG-b: biomass resource-derived ethylene glycol
- EG-fossil: fossil resource-derived ethylene glycol
- MGA: magnesium acetate
- $Sb_2O_3$: antimony trioxide
- Ti citrate: titanium citrate complex chelate
- TMPA: trimethyl phosphate
- PA: phosphoric acid
- PEP-36: bis(2,6-di-tert-butyl-4-methylphenyl) pentaerythritol-di-phosphite (Asahi Denka)
- GSY: tetrakis(2,4-di-t-butyl-5-methylphenyl) [1,1-biphenyl]-4,4'-diylbisphosphonite (Osaki Industry)
- Blue 45: Solvent Blue 45 (Clariant)
- IR1010: pentaerythritol tetrakis[3-(3,5-di-t-butyl-4-hydroxyphenyl) propionate] (IRGANOX 1010: Ciba Japan)
- $TiO_2$: titanium oxide

[Table 3]

| | Polymerization time (hrs:mins) | Polymer properties | | | | | | | | | | | | |
|---|---|---|---|---|---|---|---|---|---|---|---|---|---|---|
| | | Intrinsic viscosity IV (dl/g) | Melting point Tm(°C) | Color | | | Color difference | | Δb 290 | Carboxyl terminal group content (equivalent /ton) | Δcarboxyl terminal group 290 (equivalent /ton) | DEG content (wt%) | Phosphorus content (ppm) | Bio-based content (%) |
| | | | | L value | a value | b value | ΔE value | Corresponding fossil resource-derived polyester | | | | | | |
| Example 2-1 | 2:20 | 0.66 | 256 | 61 | -1.7 | 4.6 | 0.8 | Comparative example 2-4 | 1.4 | 21 | 5 | 1.2 | 38 | 100 |
| Example 2-2 | 3:31 | 0.66 | 245 | 54 | -0.1 | 10.1 | 9.5 | Comparative example 2-4 | 3.2 | 33 | 10 | 4.7 | 38 | 100 |
| Example 2-3 | 2:23 | 0.66 | 256 | 61 | -1.7 | 5.4 | - | - | 1.8 | 23 | 5 | 0.9 | 38 | 100 |
| Example 2-4 | 2:22 | 0.66 | 256 | 61 | -1.7 | 4.6 | 0.8 | Comparative example 2-4 | 1.6 | 23 | 5 | 0.9 | 38 | 80 |
| Example 2-5 | 2:20 | 0.66 | 256 | 61 | -1.8 | 4.8 | - | - | 2.0 | 26 | 5 | 0.9 | 50 | 100 |
| Example 2-6 | 2:17 | 0.66 | 256 | 61 | -1.7 | 4.5 | - | - | 1.4 | 21 | 5 | 0.9 | 17 | 100 |
| Example 2-7 | 2:16 | 0.66 | 256 | 61 | -1.7 | 3.8 | - | - | 1.3 | 20 | 5 | 0.9 | 8 | 100 |
| Example 2-8 | 2:18 | 0.66 | 256 | 61 | -1.9 | 7.0 | - | - | 3.1 | 26 | 8 | 0.9 | - | 100 |
| Example 2-9 | 2:13 | 0.66 | 256 | 61 | -2.2 | 5.3 | - | - | 3.0 | 30 | 10 | 0.9 | 3 | 100 |
| Example 2-10 | 2:58 | 0.66 | 256 | 59 | -2.6 | 6.9 | - | - | 1.4 | 25 | 5 | 1.6 | 180 | 100 |
| Example 2-11 | 2:20 | 0.66 | 256 | 50 | 0.1 | -1.5 | 12.3 | Comparative example 2-4 | 1.5 | 22 | 5 | 0.9 | 38 | 100 |
| Example 2-12 | 2:39 | 0.66 | 256 | 73 | -2.2 | 3.3 | - | - | 1.4 | 22 | 5 | 0.9 | 40 | 100 |
| Comparative example 2-1 | 4:00*4 | 0.36*4 | 230 | 48 | -0.1 | 17.7 | 19.1 | Comparative example 2-4 | 3.6 | 36 | 11 | 6.1 | 38 | 100 |

*4 No increase in viscosity observed after four hours of polymerization; expelled.

Abbreviations used in Table 3 are as follows.

- DEG: diethylene glycol

Example 2-13

100 parts by weight of the biomass resource-derived dimethyl terephthalate obtained in example 1-1, 61 parts by weight of biomass resource-derived ethylene glycol, and an amount of magnesium acetate equivalent to 250 ppm with respect to the obtained polymer were melted in a transesterification reactor at 150°C in a nitrogen atmosphere and heated over three hours to 250°C while stirring, a transesterification reaction was performed while maintaining the temperature at 250°C, the transesterification reaction was ended when a predetermined amount of methanol distillate was obtained, and a low-polymer polyester was obtained. The low-polymer polyester was transferred to a polymerization reaction tank.

After being transferred, the low-polymer polyester was pre-mixed in 0.05 ppm biomass resource-derived ethylene glycol in a separate mixing tank 30 minutes before the equivalent of 350 ppm of antimony trioxide with respect to the obtained polymer and 200 ppm of trimethyl phosphate with respect to the obtained polymer were added, and the mixture was stirred for 30 minutes at room temperature before being added thereto. The temperature of the polymerization reactor was maintained at 255°C, and, after five minutes, the reaction system was depressurized and a reaction begun. The temperature within the reactor was gradually increased from 255°C to 285°C and pressure was reduced to 40 Pa. The time from the beginning of the polymerization reaction until the final temperature and the final pressure were reached was 90 minutes for both. After a predetermined stirring torque was reached, the reaction system was purged of nitrogen and returned to normal pressure and the reaction was stopped, and strands of polyester were expelled from the bottom of the polymerization reactor, cooled with cooling water, and cut to obtain biomass resource-derived polyester pellets. The amount of time from the start of depressurization until the predetermined stirring torque was reached was 164 minutes. The obtained biomass resource-derived polyester had both good color and good thermal stability, and had polymer properties in no way inferior to those of a fossil resource-derived polyester produced under identical conditions (comparative example 2-2). Results are summarized in Table 5.

The obtained biomass resource-derived polyester pellets were vacuum dried for 12 hours at 150°C before being melted to a spinning temperature of 285°C, expelled from 24 spinnerets with diameter 0.18 φ, and pulled by a pulling roller at a peripheral velocity of 1,500 m/min to obtain an undrawn thread. Almost no deposits were observed around the spinnerets during spinning, and no thread breakage occurred. The obtained undrawn thread was stretch-heat treated using a hot roll drawing machine at a drawing temperature of 90°C, a heat treatment temperature of 140:C, and a stretch factor of 2.2 to obtain a 80 dtex/24 filament drawn thread. The obtained drawn thread was used to make a cylindrical knit piece and refined at 95°C, intermediate setting was performed at 160°C, Sumikaron Blue S-3RF (A) 3% owf (azo disperse dye; Sumitomo Chemtex) and an acetic acid/sodium acetate buffer were added, and dyeing was performed for 60 minutes at a bath ratio of 1:20 and a temperature of 110°C; and dye affinity was high, with a dye uptake rate of 93%.

Reference example <Mannitol titanium catalyst solution preparation>

Nitrogen substitution was performed upon a 3 L three-necked flask, and 1,000 mL ethylene glycol dehydrate (Wako Pure Chemical Industries) and 5.7 g (31.3 mmol) mannitol (TCI) were added thereto as reaction solvents and heated and stirred in an oil bath until the internal temperature reached 80°C. The mannitol dissolved after about an hour, at which point the oil bath was removed and the mixture cooled until the internal temperature reached the reaction temperature of 40°C. Once the internal temperature had reached 40°C, 10.6 (31.3 mmol) titanium tetrabutoxide (Nippon Soda) was added as a titanium compound, and a reaction was performed for 24 hours while stirring at the reaction temperature of 40°C. A colorless, transparent mannitol titanium catalyst solution (titanium content: 1.5 g/L) was thus obtained.

Example 2-14

A biomass resource-derived polyester was produced, spun, and dyed in a manner identical to example -13, except that the polymerization catalyst was changed from the mannitol titanium catalyst solution described above ("mannitol Ti" in the table) to 10 ppm as calculated in titanium atoms and an added amount of trimethyl phosphate (TMPA) as shown in Table 4. The obtained biomass resource-derived polyester had both good color and good thermal stability, and had polymer properties in no way inferior to those of a fossil resource-derived polyester produced under identical conditions (comparative example 2-3). Dye affinity and results are shown in Table 5.

Examples 2-15 and 2-16

A biomass resource-derived polyester was produced, spun, and dyed in a manner identical to example 2-13, except that the dicarboxylic acid component constituting the raw material was changed to the biomass resource-derived dimethyl terephthalates obtained in example 1-2 and example 1-3, respectively, as shown in Table 4. The obtained biomass resource-derived polyester had both good color and good thermal stability, and had polymer properties in no way inferior to those of a fossil resource-derived polyester produced under identical conditions (comparative example 2-2).
Dye affinity was also superior. Dye affinity and results are shown in Table 5.

Example 2-17

A biomass resource-derived polyester was produced, spun, and dyed in a manner identical to example 2-13, except that 500 ppm PEP-36 (Asahi Denka) with respect to the obtained polymer was added thereto as an antioxidant instead of trimethyl phosphate (TMPA). The obtained biomass resource-derived polyester had both good color and good thermal stability, and had polymer properties in no way inferior to those of a fossil resource-derived polyester produced under identical conditions (comparative example 2-5), and spinning stability and dye affinity were both good.

Example 2-18

A biomass resource-derived polyester was produced, spun, and dyed in a manner identical to example 2-15, except that 1,000 ppm pentaerythritol-tetrakis(3-laurylthiopropionate) (Sumitomo Chemical: Sumilize TP-D) with respect to the obtained polymer was added thereto as an antioxidant instead of trimethyl phosphate. The obtained biomass resource-derived polyester had both good color and good thermal stability, and had polymer properties in no way inferior to those of a fossil resource-derived polyester produced under identical conditions (comparative example 2-6), and spinning stability and dye affinity were both good.
The sulfur atom content of the obtained polyester was 98 ppm.

Example 2-19

A biomass resource-derived polyester was produced, spun, and dyed in a manner identical to example 2-13, except that 98 mol% of the biomass resource-derived dimethyl terephthalate (DMT-b) from example 1-2 and 2 mol% commercially available fossil resource-derived 5-sulfoisophthalic acid sodium salt dimethyl ester (SSIA-DM) was used as the dicarboxylic acid component. The obtained biomass resource-derived polyester had both good color and good thermal stability, and had polymer properties in no way inferior to those of a fossil resource-derived polyester produced under identical conditions (comparative example 2-7), and spinning stability and dye affinity were both good. The sulfur atom content of the obtained polyester was 2,859 ppm.

Example 2-20

A biomass resource-derived polyester was produced, spun, and dyed in a manner identical to example 2-13, except that 98 mol% of the biomass resource-derived dimethyl terephthalate (DMT-b) from example 1-4 and 2 mol% commercially available fossil resource-derived 5-sulfoisophthalic acid sodium salt dimethyl ester (SSIA-DM) was used as the dicarboxylic acid component. The obtained biomass resource-derived polyester had slightly poorer color and thermal stability than a fossil resource-derived polyester produced under identical conditions (comparative example 2-7), as well as poor spinning stability, but dye affinity was good. The sulfur atom content of the obtained polyester was 2,852 ppm.

Examples 2-21 and 2-22

A biomass resource-derived polyester was produced, spun, and dyed in a manner identical to example 2-13, except that the biomass resource-derived dimethyl terephthalates from examples 1-4 and 1-5 were used as the dicarboxylic acid components, as shown in Table 4. The obtained biomass resource-derived polyester had slightly poorer color and thermal stability than a fossil resource-derived polyester produced under identical conditions (comparative example 2-2), as well as poor spinning stability, but dye affinity was good.

[Table 4]

| | Material | | | Catalyst | | | | Additive | | | | |
|---|---|---|---|---|---|---|---|---|---|---|---|---|
| | Dicarboxylic acid component | | Diol component | Transesterification catalyst | | Polymerization catalyst | | Antioxidant | | Color adjusting agent | | $TiO_2$ |
| | | | | Type | Amount added (ppm) | Type | Amount added (ppm) | Type | Amount added (ppm) | Type | Amount added (ppm) | Amount added (wt%) |
| Example 2-13 | DMT-b | Example 1-1 | EG-b | MGA | 250 | $Sb_2O_3$ | 350 | TMPA | 200 | - | - | - |
| Example 2-14 | DMT-b | Example 1-1 | EG-b | MGA | 250 | Mannitol Ti | 10 (Ti atoms) | TMPA | 120 | - | - | - |
| Example 2-15 | DMT-b | Example 1-2 | EG-b | MGA | 250 | $Sb_2O_3$ | 350 | TMPA | 200 | - | - | - |
| Example 2-1 | DMT-b | Example 1-2 | EG-b | MGA | 620 | $Sb_2O_3$ | 300 | TMPA | 200 | - | - | - |
| Example 2-16 | DMT-b | Example 1-3 | EG-b | MGA | 250 | $Sb_2O_3$ | 350 | TMPA | 200 | - | - | - |
| Example 2-17 | DMT-b | Example 1-1 | EG-b | MGA | 250 | $Sb_2O_3$ | 350 | PEP-36 | 500 | - | - | - |
| Example 2-18 | DMT-b | Example 1-2 | EG-b | MGA | 250 | $Sb_2O_3$ | 350 | TP-D | 1000 | - | - | - |
| Example 2-19 | DMT-b | Example 1-2 | EG-b | MGA | 250 | $Sb_2O_3$ | 350 | TMPA | 200 | - | - | - |
| | SSIA-DM | commercial product | | | | | | | | | | |
| Example 2-20 | DMT-b | Example 1-4 | EG-b | MGA | 250 | $Sb_2O_3$ | 350 | TMPA | 200 | - | - | - |
| | SSIA-DM | commercial product | | | | | | | | | | |
| Example 2-21 | DMT-b | Example 1-4 | EG-b | MGA | 250 | $Sb_2O_3$ | 350 | TMPA | 200 | - | - | - |

(continued)

| | Material | | | Catalyst | | | | Additive | | | | |
|---|---|---|---|---|---|---|---|---|---|---|---|---|
| | Dicarboxylic acid component | | Diol component | Transesterification catalyst | | Polymerization catalyst | | Antioxidant | | Color adjusting agent | | $TiO_2$ |
| | | | | Type | Amount added (ppm) | Type | Amount added (ppm) | Type | Amount added (ppm) | Type | Amount added (ppm) | Amount added (wt%) |
| Example 2-22 | DMT-b | Example 1-5 | EG-b | MGA | 250 | $Sb_2O_3$ | 350 | TMPA | 200 | - | - | - |

Abbreviations used in Table 4 are as follows.

- DMT-b: biomass resource-derived dimethyl terephthalate
- SSIA-DM: 5-sulfoisophthalic acid sodium salt dimethyl ester
- EG-b: biomass resource-derived ethylene glycol
- MGA: magnesium acetate
- $Sb_2O_3$: antimony trioxide
- Mannitol Ti: mannitol titanium catalyst solution
- TMPA: trimethyl phosphate
- PEP-36: bis(2,6-di-tert-butyl-4-methylphenyl) pentaerythritol-di-phosphite (Asahi Denka)
- TP-D: pentaerythritol-tetrakis(3-laurylthiopropionate) (Sumitomo Chemical: Sumilize TP-D)

[Table 5]

| | Polymerization time (hrs:mins) | Intrinsic viscosity IV (dl/g) | Melting point Tm (°C) | Color | | | Color difference | | $\Delta$b 290 | Carboxyl terminal group content (equivalent /ton) | $\Delta$carboxyl terminal group 290 (equivalent /ton) | DEG content (wt%) | Bio-based content (%) | Dye uptake rate (%) |
|---|---|---|---|---|---|---|---|---|---|---|---|---|---|---|
| | | | | L value | a value | b value | $\Delta$E value | Corresponding fossil resource-derived polyester | | | | | | |
| Example 2-13 | 2:44 | 0.67 | 258 | 62 | -1.5 | 3.2 | 0.3 | Comparative example 2-2 | 1.1 | 25 | 5 | 0.6 | 100 | 93 |
| Example 2-14 | 2:37 | 0.67 | 258 | 65 | -1.4 | 3.5 | 0.5 | Comparative example 2-3 | 1.6 | 27 | 7 | 0.7 | 100 | 93 |
| Example 2-15 | 2:49 | 0.66 | 255 | 62 | -1.8 | 3.5 | 0.8 | Comparative example 2-2 | 1.3 | 30 | 6 | 1.8 | 100 | 96 |
| Example 2-1 | 2:20 | 0.66 | 255 | 61 | -1.7 | 4.6 | 0.8 | Comparative example 2-4 | 1.4 | 21 | 5 | 1.2 | 100 | 95 |
| Example 2-16 | 2:59 | 0.66 | 252 | 62 | -1.8 | 3.9 | 1.1 | Comparative example 2-2 | 1.6 | 32 | 7 | 2.5 | 100 | 98 |
| Example 2-17 | 2:42 | 0.67 | 258 | 63 | -1.5 | 2.6 | 0.2 | Comparative example 2-5 | 1.1 | 23 | 5 | 0.6 | 100 | 93 |
| Example 2-18 | 2:47 | 0.67 | 256 | 62 | -1.7 | 3.9 | 0.8 | Comparative example 2-6 | 1.4 | 31 | 5 | 1.8 | 100 | 96 |
| Example 2-19 | 2:31 | 0.65 | 252 | 61 | -1.6 | 9.8 | 0.7 | Comparative example 2-7 | 1.8 | 38 | 7 | 2.1 | 98 | 97 |
| Example 2-20 | 2:35 | 0.65 | 252 | 59 | -1.8 | 13.2 | 4.6 | Comparative example 2-7 | 3.5 | 42 | 12 | 3.5 | 98 | 99 |
| Example 2-21 | 2:55 | 0.67 | 252 | 60 | -1.8 | 8.5 | 6.0 | Comparative example 2-2 | 3.1 | 38 | 13 | 3.1 | 100 | 98 |
| Example 2-22 | 3:15 | 0.67 | 252 | 60 | -1.9 | 10.3 | 7.7 | Comparative example 2-2 | 3.6 | 43 | 15 | 3.5 | 100 | 99 |

Abbreviations used in Table 5 are as follows.

- DEG: diethylene glycol

Comparative example 2-2

A biomass resource-derived polyester was produced, spun, and dyed in a manner identical to example 2-13, except that fossil resource-derived ethylene glycol and dimethyl terephthalate were used as the diol component and dicarboxylic acid component.

Comparative example 2-3

A biomass resource-derived polyester was produced, spun, and dyed in a manner identical to example 2-14, except that fossil resource-derived ethylene glycol and dimethyl terephthalate were used as the diol component and dicarboxylic acid component.

Comparative example 2-4

A biomass resource-derived polyester was produced, spun, and dyed in a manner identical to example 2-1, except that fossil resource-derived ethylene glycol and dimethyl terephthalate were used as the diol component and dicarboxylic acid component.

Comparative examples 2-5 and 2-6

Biomass resource-derived polyesters were produced, spun, and dyed in a manner identical to examples 2-17 and 2-18, respectively, except that fossil resource-derived ethylene glycol and dimethyl terephthalate were used as the diol component and dicarboxylic acid component. The sulfur atom content of the polyester obtained in comparative example 2-6 was 93 ppm.

Comparative example 2-7

A fossil resource-derived polyester was produced, spun, and dyed in a manner identical to example 2-19, except that fossil resource-derived ethylene glycol (EG-fossil) was used as the diol component and 98 mol% fossil resource-derived dimethyl terephthalate (DMT-fossil) as the dicarboxylic acid component, as well as 2 mol% commercially available fossil resource-derived 5-sulfoisophthalic acid sodium salt dimethyl ester (SSIA-DM). The sulfur atom content of the obtained polyester was 2,845 ppm.

The above comparative examples 2-2 through 2-7 all had good spinning stability. Evaluation results for polymer properties and dye affinity are shown in Table 7.

[Table 6]

| | Material | | | Catalyst | | | | Additive | | | | |
|---|---|---|---|---|---|---|---|---|---|---|---|---|
| | Diol component | Dicarboxylic acid component | | Transesterification catalyst | | Polymerization catalyst | | Antioxidant | | Color adjusting agent | | $TiO_2$ |
| | | | | Type | Amount added (ppm) | Type | Amount added (ppm) | Type | Amount added (ppm) | Type | Amount added (ppm) | Amount added (wt%) |
| Comparative example 2-2 | EG-fossil | DMT-fossil | Comparative example 1-2 | MGA | 250 | $Sb_2O_3$ | 350 | TMPA | 200 | - | - | - |
| Comparative example 2-3 | EG-fossil | DMT-fossil | Comparative example 1-2 | MGA | 250 | Mannitol Ti | 10 (Ti atoms) | TMPA | 120 | - | - | - |
| Comparative example 2-4 | EG-fossil | DMT-fossil | Comparative example 1-2 | MGA | 620 | Sb2O3 | 300 | TMPA | 200 | - | - | - |
| Comparative example 2-5 | EG-fossil | DMT-fossil | Comparative example 1-2 | MGA | 250 | $Sb_2O_3$ | 350 | PEP-35 | 500 | - | - | - |
| Comparative example 2-6 | EG-fossil | DMT-fossil | Comparative example 1-2 | MGA | 250 | $Sb_2O_3$ | 350 | TP-D | 1000 | - | - | - |
| Comparative example 2-7 | EG-fossil | DMT-fossil | Comparative example 1-2 | MGA | 250 | $Sb_2O_3$ | 350 | TMPA | 200 | - | - | - |
| | | SSIA-DM | commercial product | | | | | | | | | |

Abbreviations used in Table 6 are as follows.

- EG-fossil: fossil resource-derived ethylene glycol
- DMT-fossil: fossil resource-derived dimethyl terephthalate
- SSIA-DM: 5-sulfoisophthalic acid sodium salt dimethyl ester
- MGA: magnesium acetate
- $Sb_2O_3$: antimony trioxide
- Mannitol Ti: mannitol titanium catalyst solution
- TMPA: trimethyl phosphate
- PEP-36: bis(2,6-di-tert-butyl-4-methylphenyl) pentaerythritol-di-phosphite (Asahi Denka)
- TP-D: pentaerythritol-tetrakis(3-laurylthiopropionate) (Sumitomo Chemical: Sumilize TP-D)

[Table 7]

| | Polymerization time (hrs:mins) | Intrinsic viscosity IV (dl/g) | Melting point Tm (°C) | Color | | | Color difference | | Δb 290 | Carboxyl terminal group content (equivalent /ton) | Δcarboxyl terminal group 290 (equivalent /ton) | DEG content (wt%) | Bio-based content (%) | Dye uptake rate (%) |
|---|---|---|---|---|---|---|---|---|---|---|---|---|---|---|
| | | | | L value | a value | b value | ΔE value | Corresponding fossil resource-derived polyester | | | | | | |
| Comparative example 2-2 | 2:42 | 0.67 | 258 | 62 | -1.4 | 2.9 | - | - | 1.1 | 27 | 5 | 0.5 | 0 | 92 |
| Comparative example 2-3 | 2:35 | 0.67 | 258 | 65 | -1.4 | 3.1 | - | - | 1.5 | 28 | 7 | 0.5 | 0 | 92 |
| Comparative example 2-4 | 2:29 | 0.66 | 258 | 61 | -1.5 | 3.8 | - | - | 1.2 | 20 | 5 | 0.6 | 0 | 93 |
| Comparative example 2-5 | 2:40 | 0.67 | 258 | 63 | -1.4 | 2.4 | - | - | 1.1 | 22 | 5 | 0.6 | 0 | 93 |
| Comparative example 2-6 | 2:41 | 0.67 | 258 | 62 | -1.4 | 3.2 | - | - | 1.1 | 30 | 5 | 0.6 | 0 | 93 |
| Comparative example 2-7 | 2:25 | 0.66 | 254 | 61 | -1.4 | 9.1 | - | - | 1.7 | 34 | 7 | 0.9 | 0 | 93 |

Abbreviations used in Table 7 are as follows.

- DEG: diethylene glycol

## Claims

1. A method of producing a biomass resource-derived polyester using a biomass resource-derived dicarboxylic acid and/or ester forming derivative thereof, the method **characterized in that** the biomass resource-derived dicarboxylic acid and/or ester forming derivative thereof fulfills at least one of conditions (A), (B), and (C) described below.

   (A) The amount of potassium hydroxide needed to neutralize an acid component extracted using an organic solvent from 1 g of the dicarboxylic acid and/or ester forming derivative thereof is 0.1 mg KOH/g or less
   (B) The amount of potassium hydroxide needed to neutralize an acid component extracted using water from 1 g of the dicarboxylic acid and/or ester forming derivative thereof is 0.05 mg KOH/g or less
   (C) Sulfuric acid ion content is 40 ppm or less

2. The method of producing a polyester according to claim 1, **characterized in that** the biomass resource-derived dicarboxylic acid and/or ester forming derivative thereof has an ash content of 100 ppm or less.

3. The method of producing a biomass resource-derived polyester according to claim 1 or claim 2, **characterized in** using a biomass resource-derived diol.

4. The method of producing a biomass resource-derived polyester according to any one of claims 1 through 3, **characterized in** using an antioxidant.

5. A method of producing a biomass resource-derived polyester according to the present invention, the method **characterized in** using a biomass resource-derived dicarboxylic acid and/or ester forming derivative thereof obtained by a process including steps (1) through (4) described below.

   (1) Obtaining isobutanol from a biomass resource by means of a fermentation method
   (2) Obtaining isobutene from isobutanol by means of dehydration
   (3) Converting isobutene to paraxylene
   (4) Obtaining a terephthalic acid and/or ester forming derivative thereof from paraxylene

6. The biomass resource-derived polyester obtained using the production method according to any one of claims 1 through 5, the biomass resource-derived polyester having an intrinsic viscosity of from 0.4 to 2.0 $dlg^{-1}$.

7. The biomass resource-derived polyester obtained using the production method according to any one of claims 1 through 5, the biomass resource-derived polyester having a color value b of from -10 to 15.

8. The biomass resource-derived polyester obtained using the production method according to any one of claims 1 through 5, the biomass resource-derived polyester having a diethylene glycol content of from 0.1 to 3.0% by weight.

9. A biomass resource-derived dicarboxylic acid and/or ester forming derivative thereof fulfilling at least one of the following conditions (A), (B), and (C).

   (A) The amount of potassium hydroxide needed to neutralize an acid component extracted using an organic solvent from 1 g of the dicarboxylic acid and/or ester forming derivative thereof is 0.1 mg KOH/g or less
   (B) The amount of potassium hydroxide needed to neutralize an acid component extracted using water from 1 g of the dicarboxylic acid and/or ester forming derivative thereof is 0.05 mg KOH/g or less
   (C) Sulfuric acid ion content is 40 ppm or less

10. The biomass resource-derived dicarboxylic acid and/or ester forming derivative thereof according to claim 9, **characterized in** having an ash content of 100 ppm.

11. The method of producing a biomass resource-derived dicarboxylic acid and/or ester forming derivative thereof according to claim 9 or claim 10, **characterized in that** a dialkyl ester of the dicarboxylic acid is refined by distillation.

**INTERNATIONAL SEARCH REPORT**

International application No.
PCT/JP2012/065320

A. CLASSIFICATION OF SUBJECT MATTER
*C08G63/183*(2006.01)i, *C07C69/82*(2006.01)i

According to International Patent Classification (IPC) or to both national classification and IPC

B. FIELDS SEARCHED

Minimum documentation searched (classification system followed by classification symbols)
C08G63/183, C07C69/82

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2006-328371 A (Mitsubishi Chemical Corp.), 07 December 2006 (07.12.2006), paragraphs [0006], [0021], [0035], [0092] (Family: none) | 6-10<br>1-4,11 |
| Y | JP 2004-2515 A (Mitsubishi Chemical Corp.), 08 January 2004 (08.01.2004), paragraph [0009] (Family: none) | 1-4,11 |
| Y | JP 2006-316095 A (Teijin Fibers Ltd.), 24 November 2006 (24.11.2006), paragraph [0015] (Family: none) | 1-4,11 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 August, 2012 (28.08.12) | 04 September, 2012 (04.09.12) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No.
PCT/JP2012/065320

C (Continuation). DOCUMENTS CONSIDERED TO BE RELEVANT

| Category* | Citation of document, with indication, where appropriate, of the relevant passages | Relevant to claim No. |
|---|---|---|
| X | WO 2009/79213 A2 (GEVO, INC.), 25 June 2009 (25.06.2009), examples 1, 2, 9, 17, 58 & JP 2011-505490 A & US 2009/0299109 A1 & EP 2225351 A2 | 5 |
| P,A | JP 2011-219736 A (Toray Industries, Inc.), 04 November 2011 (04.11.2011), entire text (Family: none) | 1-11 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6428767 B1
- CN 101046007
- JP 2009091694 A
- WO 2009072462 PCT
- WO 2010078328 A
- WO 2009120457 A
- JP 2011219736 A
- WO 2010148070 A
- JP 2007176873 A
- WO 2009064515 A
- WO 2009079213 PCT
- WO 2010151346 PCT
- JP 2011168501 A
- JP 2009079213 PCT
- WO 2009079213 A